# EUROPEAN PATENT APPLICATION

(11) **EP 2 426 134 A2**
(43) Date of publication of application: **07.03.2012**
(21) Application number: 11006568.7
(22) Date of filing: 22.11.2006
(51) Int. Cl.: C07D 495/04, A61K 31/4365, A61P 29/00, C07D 403/04

(54) **Method of modulating stress-activated protein kinase system**

(30) Priority: 23.11.2005 US 739315 P; 21.02.2006 US 775823 P; 20.04.2006 US 793526 P
(62) Divisional of application: 06844534.5
(71) Applicant: Intermune, Inc., Brisbane, CA 94005 (US)
(72) Inventor: Seiwert, Scott D., Pacifica, California 94044 (US); Kossen, Karl, Brisbane, California 94005 (US); Serebryany, Vladimir, Burlingame, California 94010 (US)
(74) Representative: Pilkington, Stephanie Joan

(57) **Abstract**

It has now been discovered that a high therapeutic effect in treating various disorders associated with enhanced activity of kinase p38 may be achieved by using a potent p38? kinase inhibitor compound which also has inhibitory activity against p38?. Furthermore, reducing the activities of both kinase p38? and kinase p38? without reducing the activity of a kinase p38? to such an extent that undesired side effects are observed upon administration to a subject having a disorder associated with enhanced activity of kinase p38 has been discovered to be achievable by modifying inhibitors of p38? such that the modification engenders inhibitory activity against p38?. Described are compounds with activity against p38? and p38?.; Disclosed are methods of using described compounds and compositions to modulate a stress activated protein kinase (SAPK) system with an active compound, wherein the active compound exhibits inhibition of the p38? and p38? MAPKs. Also disclosed are methods for identifying compounds which inhibit p38? and p38? MAPKs and which can modulate a stress activated protein kinase (SAPK) system.

SEQUENCE LISTING

<110> InterMune, Inc.

<120> METHOD OF MODULATING STRESS-ACTIVATED
  PROTEIN KINASE SYSTEM

<130> INTMU.028VPC

<150> 60/793,526
<151> 2006-04-20

<150> 60/775,823
<151> 2006-02-21

<150> 60/739,315
<151> 2005-11-23

<160> 8

<170> FastSEQ for Windows Version 4.0

<210> 1
<211> 57
<212> DNA
<213> Artificial Sequence

<220>
<223> Chemically Synthesized

<400> 1
   ccggctggat gacttcacgg actttctcga gaaagtccgt gaagtcatcc agttttt    57

<210> 2
<211> 57
<212> DNA
<213> Artificial Sequence

<220>
<223> Chemically Synthesized

<400> 2
   ccggcatctt gaattggatg cgctactcga gtagcgcatc caattcaaga tgttttt    57

<210> 3
<211> 57
<212> DNA
<213> Artificial Sequence

<220>
<223> Chemically Synthesized

<400> 3
   ccggccaggt ccagaagtat gatgactcga gtcatcatac ttctggacct ggttttt    57

<210> 4
<211> 57
<212> DNA
<213> Artificial Sequence

<220>
<223> Chemically Synthesized

<400> 4
   ccgggccctt ccagtccgag ctgttctcga gaacagctcg gactggaagg gcttttt    57

<210> 5
<211> 57
<212> DNA
<213> Artificial Sequence

<220>
<223> Chemically Synthesized

<400> 5
   ccgggttacg tgtggcagtg aagaactcga gttcttcact gccacacgta acttttt 57

<210> 6
<211> 57
<212> DNA
<213> Artificial Sequence

<220>
<223> Chemically Synthesized

<400> 6
   ccgggttcag ttccttatct accaactcga gttggtagat aaggaactga acttttt 57

<210> 7
<211> 57
<212> DNA
<213> Artificial Sequence

<220>
<223> Chemically Synthesized

<400> 7
   ccgggacata attcacaggg acctactcga gtaggtccct gtgaattatg tcttttt 57

<210> 8
<211> 57
<212> DNA
<213> Artificial Sequence

<220>
<223> Chemically Synthesized

<400> 8
   ccggctcggc acacagatga tgaaactcga gtttcatcat ctgtgtgccg agttttt 57

## Description

### Field of the Invention

This invention relates to compounds and methods useful in treating various fibrotic conditions, including those associated with enhanced activity of kinase p38.

### Background of the Invention

A large number of chronic and acute conditions have been recognized to be associated with perturbation of the inflammatory response. It is believed that numerous cytokines participate in this response, including IL-1, IL-6, IL-8 and TNFα. It appears that the activity of these cytokines in the regulation of inflammation may be associated with the activation of an enzyme on the cell signaling pathway, a member of the MAP kinase family generally known as p38 and also known as SAPK, CSBP and RK.

Several inhibitors of p38, such as NPC 31169, SB239063, SB203580, FR-167653, and pirfenidone have been tested *in vitro* and/or *in vivo* and found to be effective for modulating inflammatory responses.

There continues to be a need for safe and effective drugs to treat various inflammatory conditions such as inflammatory pulmonary fibrosis.

### Summary of the Invention

In one embodiment, there is provided a method of modulating a stress activated protein kinase (SAPK) system, comprising contacting a compound with a p38 mitogen-activated protein kinase (MAPK),
wherein the compound exhibits an IC₅₀ in the range of about 10 pM to about 500 µM for inhibition of the p38γ; and
wherein the compound exhibits an IC₅₀ for inhibition of the p38α MAPK which is at least twofold, fivefold, or tenfold higher than the IC₅₀ for inhibition of the p38γ MAPK.
In another embodiment, there is provided a method of modulating a stress activated protein kinase (SAPK) system, comprising contacting a compound with a p38 mitogen-activated protein kinase (MAPK),
wherein the compound exhibits an IC₅₀ value in the range of about 10 pM to about 500 µM for inhibition of the p38γ MAPK; and
wherein the compound exhibits an IC₅₀ value in the range of about 10 pM to about 1500 µM for inhibition of the p38α MAPK.

In another embodiment, there is provided a method of treating or preventing a disease state in a subject, comprising
identifying a subject at risk for or having a fibrotic condition; administering a compound to the subject in an effective amount to treat or prevent the fibrotic condition;
wherein the compound exhibits an IC₅₀ in the range of about 10 pM to about 500 µM for inhibition of p38y; and
wherein the compound exhibits an IC₅₀ for inhibition of the p38α MAPK which is at least twofold, fivefold, or tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38α MAPK; and
assaying a plurality of compounds from the library for inhibition of a p38γ MAPK; and
selecting at least one compound from the plurality of compounds, wherein the compound exhibits an IC₅₀ in the range of about 10 pM to about 500 µM for inhibition of the p38γ MAPK; and
wherein the compound exhibits an IC₅₀ for inhibition of the p38α MAPK which is at least twofold, fivefold, or tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38γ MAPK; and
selecting at least one compound from the plurality of compounds, wherein the compound exhibits an IC₅₀ in the range of about 10 pM to about 500 µM for inhibition of the p38γ MAPK; and
wherein the compound exhibits an IC₅₀ for inhibition of the p38α MAPK which is at least twofold, fivefold, or tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38α MAPK; and
selecting at least one compound from the plurality of compounds, wherein the compound exhibits an IC₅₀ in the range of about 10 pM to about 1500 µM for inhibition of the p38α MAPK; and
wherein the compound exhibits an IC₅₀ for inhibition of the p38α MAPK which is at least twofold, fivefold, or tenfold higher than the IC₅₀ for inhibition of the p38γ MAPK.

In one embodiment, there is provided a method of modulating a stress activated protein kinase (SAPK) system, comprising contacting a compound with a p38 mitogen-activated protein kinase (MAPK),
wherein the compound exhibits an IC₅₀ in the range of about 100 µM to about 1000 µM for inhibition of the p38γ; and
wherein the compound exhibits an IC₅₀ for inhibition of the p38α MAPK which is at least twofold higher than the IC₅₀ for inhibition of the p38γ MAPK.

In another embodiment, there is provided a method of treating or preventing a disease state in a subject, comprising
identifying a subject at risk for or having a fibrotic condition; administering a compound to the subject in an effective amount to treat or prevent the fibrotic condition;
wherein the compound exhibits an IC₅₀ in the range of about 100 µM to about 1000 µM for inhibition of p38y; and
wherein the compound exhibits an IC₅₀ for inhibition of the p38α MAPK which is at least tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38α MAPK; and
assaying a plurality of compounds from the library for inhibition of a p38γ MAPK; and
selecting at least one compound from the plurality of compounds, wherein the compound exhibits an IC₅₀ in the range of about 10 pM to about 5 µM for inhibition of the p38γ MAPK; and
wherein the compound exhibits an IC₅₀ for inhibition of the p38α MAPK which is at least tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38γ MAPK; and
selecting at least one compound from the plurality of compounds, wherein the compound exhibits an IC₅₀ in the range of about 10 pM to about 5 µM for inhibition of the p38γ MAPK; and
wherein the compound exhibits an IC₅₀ for inhibition of the p38α MAPK which is at least tenfold higher than the IC₅₀ for inhibition of the p38γ MAPK.

In another embodiment, there is provided a method of identifying a pharmaceutically active compound, comprising:
providing a library of compounds;
assaying a plurality of compounds from the library for inhibition of a p38α MAPK; and
selecting at least one compound from the plurality of compounds, wherein the compound exhibits an IC₅₀ in the range of about 10 pM to about 5 µM for inhibition of the p38γ MAPK; and
wherein the compound exhibits an IC₅₀ for inhibition of the p38α MAPK which is at least tenfold higher than the IC₅₀ for inhibition of the p38γ MAPK.

In some of the above embodiments, the compound exhibits an IC₅₀ in the range of about 10 pM to about 5 µM for inhibition of the p38γ MAPK. In other embodiments, the compound exhibits an IC₅₀ in the range of about 100 nM to about 500 µM for inhibition of the p38γ MAPK.

In some of the above embodiments, the compound is not selected from the group consisting of: 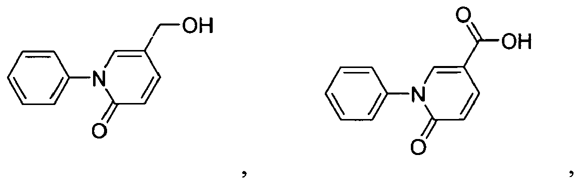 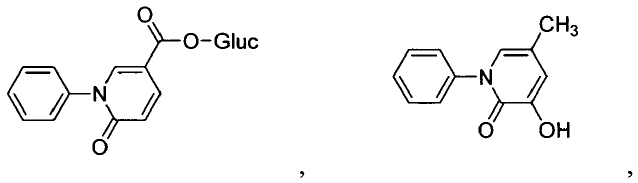 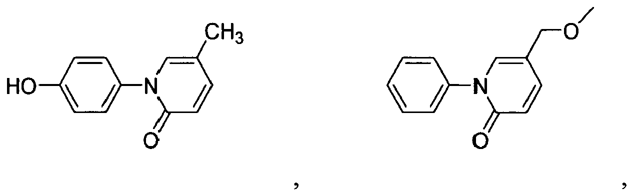 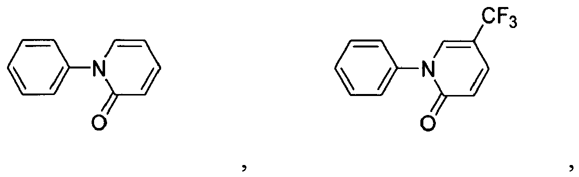 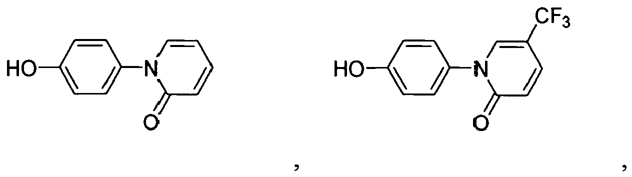 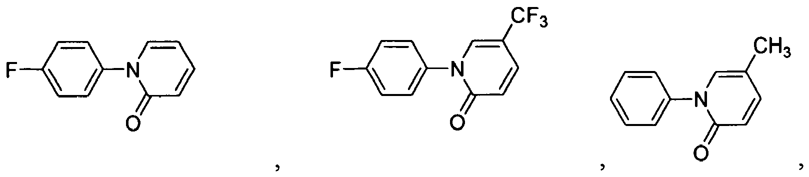 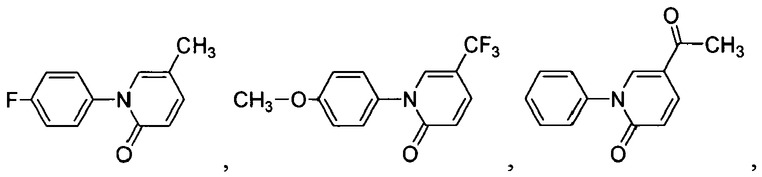 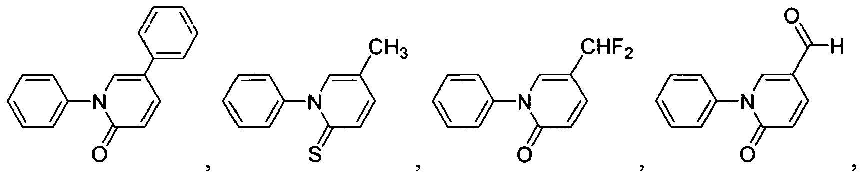 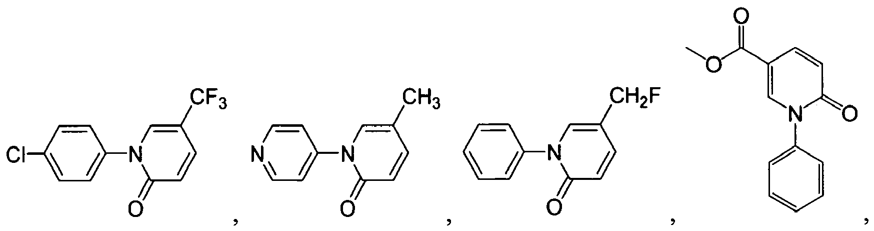 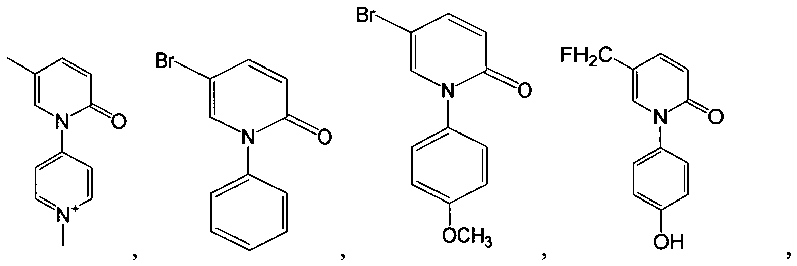 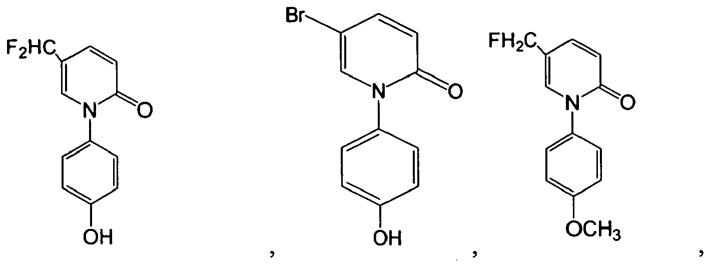 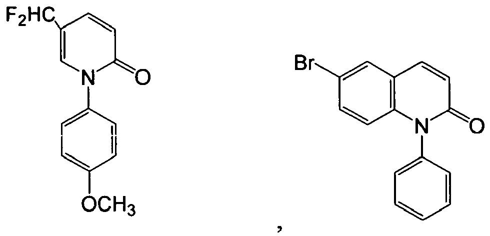 , and pirfenidone.

In another embodiment, there is provided a compound having the formula of Genus I:

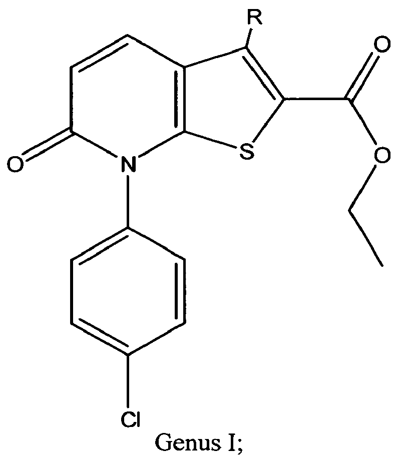

wherein

R is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C₆ or ₁₀ aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;

or a pharmaceutically acceptable salt, ester, solvate or prodrug of the compound.

In an embodiment, the compound of Genus I exhibits an IC₅₀ in the range of about 10 pM to about 5 µM for inhibition of p38γ.

Another embodiment provides a compound having the formula of Genus II:

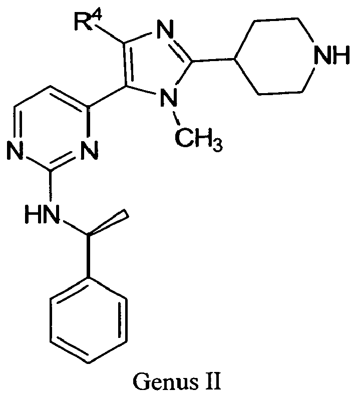

wherein

R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;

or a pharmaceutically acceptable salt, ester, solvate or prodrug of the compound.

In an embodiment, the compound of Genus II exhibits an IC₅₀ in the range of about 10 pM to about 5 µM for inhibition of p38γ.

In another embodiment, there is provided a compound having the formula of Genus III:

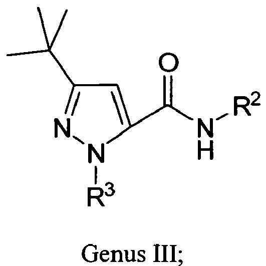

wherein

R² is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl; and

R³ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;

or a pharmaceutically acceptable salt, ester, solvate or prodrug of the compound.

In an embodiment, the compound of Genus III exhibits an IC₅₀ in the range of about 10 pM to about 5 µM for inhibition of p3 8y.

In one embodiment, the compound of Genus III is not 1-(5-tert-Butyl-2-p-tolyl-2H-pyrazol-3-yl)-3-[4-(2-morpholin-4-yl-ethoxy)naph- thalen-1-yl]urea (BIRB 796).

Another embodiment provides a compound having the formula of Genus IV:

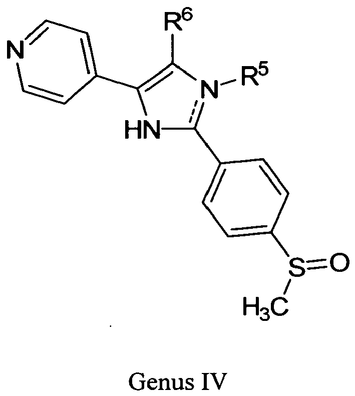

wherein

R⁵ and R⁶ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;

or a pharmaceutically acceptable salt, ester, solvate or prodrug of the compound.

In an embodiment, the compound of Genus IV exhibits an IC₅₀ in the range of about 10 pM to about 5 µM for inhibition of p3 8y.

In one embodiment, the compound of Genus IV is not 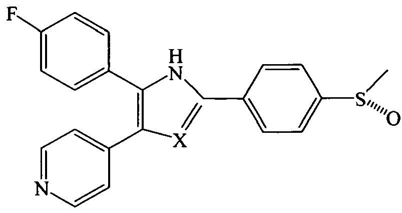 where X is N.

These and other embodiments are described in greater detail below.

### Description of the Drawings

Figure 1 depicts the level of collagen in uninduced cells as a function of different lentiviral particles, including various shRNA-encoding lentiviral particles, administered to the cells.

Figure 2 depicts the level of collagen in cells induced with TGF-β as a function of different lentiviral particles, including various shRNA-encoding lentiviral particles, administered to the cells.

### Detailed Description of the Preferred Embodiments

It has now been discovered that a high therapeutic effect in treating various disorders associated with enhanced activity of kinase p38 may be achieved by using a potent p38γ kinase inhibitor compound which also has inhibitory activity against p38α. Furthermore, reducing the activities of both kinase p38γ and kinase p38α without reducing the activity of a kinase p38α to such an extent that undesired side effects are observed upon administration to a subject having a disorder associated with enhanced activity of kinase p38 has been discovered to be achievable by modifying inhibitors of p38α such that the modification engenders inhibitory activity against p38γ.

Therefore, in one embodiment there is provided a method of modulating a stress-activated kinase (SAPK) system by contacting a compound with a p38 mitogen-activated protein kinase (MAPK). A preferred compound exhibits an IC₅₀ against a p38γ MAPK in the range of about 10 pM to about 5 µM, preferably about 50 pM to about 1 µM for the inhibition of p38γ MAPK, more preferably about 50 pM to about 200 nM for the inhibition of p38γ MAPK. The preferred compound also exhibits an IC₅₀ value against p38α which is not less than twice its IC₅₀ value against p38γ.

"Mitogen-activated protein kinases (MAPKs)" are evolutionarily conserved serine/threonine kinases involved in the regulation of many cellular events. Several MAPK groups have been identified in mammalian cells, including extracellular signal-regulated kinase (ERK), p38, and SAPK/JNK. It is believed that MAPKs are activated by their specific MAPK kinases (MAPKKs): ERK by MEK1 and MEK2, p38 by MKK3 and MKK6, and SAPK/JNK by SEK1 (also known as MKK4) and MKK7 (SEK2). These MAPKKs may also be activated by various MAPKK kinases (MAPKKKs) such as Raf, MLK, MEKK1, TAK1, and ASK1.

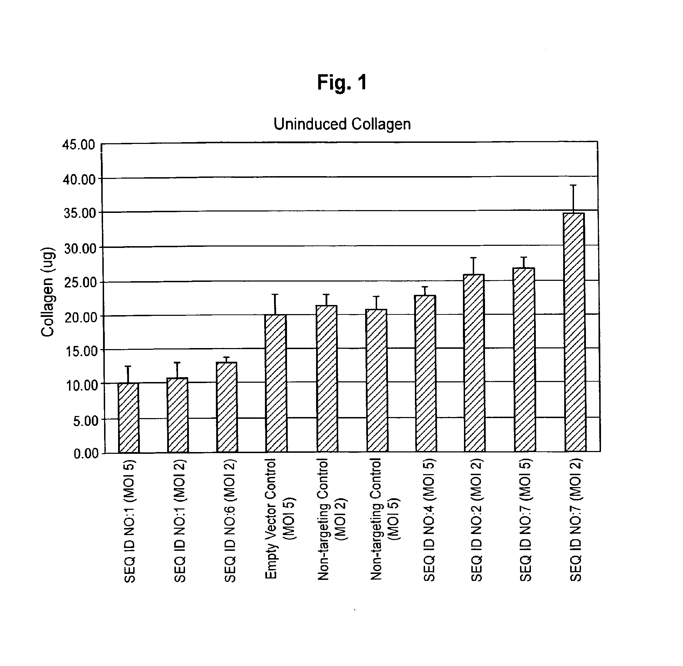

It is believed that the MAPK network involves at least twelve cloned highly conserved, proline-directed serine-threonine kinases which, when activated by cell stresses (oxidative stress, DNA damage, heat or osmotic shock, ultraviolet irradiation, ischemia-reperfusion), exogenous agents (anisomycin, Na arsenite, lipopolysaccharide, LPS) or pro-inflammatory cytokines, TNF-α and IL-1β, can phosphorylate and activate other kinases or nuclear proteins such as transcription factors in either the cytoplasm or the nucleus (see Figure 1 from Underwood et al. 2001 Prog Respir Res 31:342-345).

### p38 MAPK

As used herein, "p38 MAPK" is a member of the stress-activated protein kinase family, which includes at least 4 isoforms (α, β, γ, δ), several of which are considered important in processes critical to the inflammatory response and tissue remodeling (Lee et al. 2000 Immunopharmacol. 47:185-201). The predominant kinases in monocytes and macrophages, p38α and p38β, appear more widely expressed compared to p38γ (skeletal muscle) or p38δ (testes, pancreas, prostate, small intestine, and in salivary, pituitary and adrenal glands). A number of substrates of p38 MAP kinase have been identified including other kinases (MAPKAP K2/3, PRAK, MNK 1/2, MSK1/RLPK, RSK-B), transcription factors (ATF2/6, myocyte enhancer factor 2, nuclear transcription factor-β, CHOP/GADD153, Elk1 and SAP-1A1) and cytosolic proteins (stathmin), many of which are important physiologically.

Jiang, Y. et al. 1996 J Biol Chem 271:17920-17926 reported characterization of p38β as a 372-amino acid protein closely related to p38-α. Both p38α and p38β are activated by proinflammatory cytokines and environmental stress, p38β is preferentially activated by MAP kinase kinase-6 (MKK6) and preferentially activated transcription factor 2. Kumar, S. et al. 1997 Biochem Biophys Res Comm 235:533-538 and Stein, B. et al. 1997 J Biol Chem 272:19509-19517 reported a second isoform of p38β, p-38β2, containing 364 amino acids with 73% identity to p38α. It is believed that p38β is activated by proinflammatory cytokines and environmental stress, although the second reported p38β isoform, p38β2, appears to be preferentially expressed in the central nervous system (CNS), heart and skeletal muscle, compared to the more ubiquitous tissue expression of p38α. Furthermore, it is believed that activated transcription factor-2 (ATF-2) is a better substrate for p38β2 than for p38α.

The identification of p38γ was reported by Li, Z. et al. 1996 Biochem Biophys Res Comm 228:334-340 and of p38δ by Wang, X. et al. 1997 J Biol Chem 272:23668-23674 and by Kumar, S. et al. 1997 Biochem Biophys Res Comm 235:533-538. These two p38 isoforms (γ and δ) represent a unique subset of the MAPK family based on their tissue expression patterns, substrate utilization, response to direct and indirect stimuli, and susceptibility to kinase inhibitors. It is believed that p38α and β are closely related, but diverge from γ and δ, which are more closely related to each other.

Typically the p38 MAP kinase pathway is directly or indirectly activated by cell surface receptors, such as receptor tyrosine kinases, chemokine or G protein-coupled receptors, which have been activated by a specific ligand, e.g., cytokines, chemokines or lipopolysaccharide (LPS) binding to a cognate receptor. Subsequently, p38 MAP kinase is activated by phosphorylation on residues threonine 180 and tyrosine 182. After activation, p38 MAP kinase can phosphorylate other intracellular proteins, including protein kinases, and can be translocated to the cell nucleus, where it phosphorylates and activates transcription factors leading to the expression of proinflammatory cytokines and other proteins that contribute to the inflammatory response, cell adhesion, and proteolytic degradation. For example, in cells of myeloid lineage, such as macrophages and monocytes, both IL-1β and TNFα are transcribed in response to p38 activation. Subsequent translation and secretion of these and other cytokines initiates a local or systemic inflammatory response in adjacent tissue and through infiltration of leukocytes. While this response is a normal part of physiological responses to cellular stress, acute or chronic cellular stress leads to the excess, unregulated, or excess and unregulated expression of pro-inflammatory cytokines. This, in turn, leads to tissue damage, often resulting in pain and debilitation.

In alveolar macrophages, inhibition of p38 kinases with p38 inhibitor, SB203580, reduces cytokine gene products. It is believed that inflammatory cytokines (TNF-α, IFN-γ, IL-4, IL-5) and chemokines (IL-8, RANTES, eotaxin) are capable of regulating or supporting chronic airway inflammation. The production and action of many of the potential mediators of airway inflammation appear to be dependent upon the stress-activated MAP kinase system (SAPK) or p38 kinase cascade (Underwood et al. 2001 Prog Respir Res 31:342-345). Activation of the p38 kinase pathway by numerous environmental stimuli results in the elaboration of recognized inflammatory mediators whose production is considered to be translationarily regulated. In addition, a variety of inflammatory mediators activate p38 MAPK which may then activate downstream targets of the MAPK system including other kinases or transcription factors, thus creating the potential for an amplified inflammatory process in the lung.

### Downstream substrates of p38 group of MAP kinases

Protein kinase substrates of p38α or p38β: MAP kinase-activated protein kinase 2 (MAPKAPK2 or M2), MAP kinase interaction protein kinase (MNK1), p38 regulated/activated kinase (PRAK), mitogen- and stress-activated kinase (MSK: RSK-B or RLPK).

Transcription factors activated by p38: activating transcription factor (ATF)-1, 2 and 6, SRF accessory protein 1 (Sap 1), CHOP (growth arrest and DNA damage inducible gene 153, or GADD153), p53, C/EBPβ, myocyte enhance factor 2C (MEF2C), MEF2A, MITF1, DDIT3, ELK1, NFAT, and high mobility group-box protein (HBP1).

Other types of substrates for p38: cPLA2, Na⁺/H⁺ exchanger isoform-1, tau, keratin 8, and stathmin.

Genes regulated by the p38 pathway: c-jun, c-fos, junB, IL-1, TNF, IL-6, IL-8, MCP-1, VCAM-1, iNOS, PPARγ, cyclooxygenase (COX)-2, collagenase-1 (MMP-1), Collagenase-3 (MMP-13), HIV-LTR, Fgl-2, brain natriuretic peptide (BNP), CD23, CCK, phosphoenolpyruvate carboxy-kinase-cytosolic, cyclin D1, LDL receptor (Ono et al. 2000 Cellular Signalling 12:1-13).

### Biological consequences of p38 activation

### p38 and inflammation

Acute and chronic inflammation are believed to be central to the pathogenesis of many diseases such as rheumatoid arthritis, asthma, chronic obstructive pulmonary disease (COPD) and acute respiratory distress syndrome (ARDS). The activation of the p38 pathway may play an central role in: (1) production of proinflammatory cytokines such as IL-1β, TNF-α and IL-6; (2) induction of enzymes such as COX-2, which controls connective tissue remodeling in pathological condition; (3) expression of an intracellular enzyme such as iNOS, which regulates oxidation; (4) induction of adherent proteins such as VCAM-1 and many other inflammatory related molecules. In addition to these, the p38 pathway may play a regulatory role in the proliferation and differentiation of cells of the immune system. p38 may participate in GM-CSF, CSF, EPO, and CD40-induced cell proliferation and/or differentiation.

The role of the p38 pathway in inflammatory-related diseases was studied in several animal models. Inhibition of p38 by SB203580 reduced mortality in a murine model of endotoxin-induced shock and inhibited the development of mouse collagen-induced arthritis and rat adjuvant arthritis. A recent study showed that SB220025, which is a more potent p38 inhibitor, caused a significant dose-dependent decrease in vascular density of the granuloma. These results indicate that p38 or the components of the p38 pathway can be a therapeutic target for inflammatory disease.

### p38 and fibrosis

Uncontrolled and/or aberrant deposition of extracellular matrix proteins leads to fibrosis and is fundamental to the pathogenesis of diseases including idiopathic pulmonary fibrosis (IPF), sarcoidosis, chronic obstructive pulmonary disease (COPD), and cirrhosis. p38 has been implicated in several pro-fibrotic pathways events through its role in TGF-β receptor signaling (Kaminska et al Acta Biochimica Polonica 52(2): 329-37). TGF-β is fundamental to fibrosis and is involved in events including induction of collagen deposition, synthesis of pro-fibrotic cytokines, fibroblast proliferation, myofibroblast differentiation, and epithelial-mesenchymal transisition (Kaminska et al Acta Biochimica Polonica 52(2): 329-37; Border and Noble 1994 NEJM 331: 1286-92) (R. Newton and N.S. Holden, Drug Discovery Today: Disease Mechanisms, Volume 3, Issue 1, Spring 2006, Pages 53-61). p38 is activated by additional cytokines and growth factors that may be associated with fibrosis including: interleukin-1β (IL-1β), interleukin-17 (IL-17), epidermal growth factor (EGF), and platelet derived growth factor (PDGF).

Fibrosis, for example in liver, occurs by excessive deposition of extracellular matrix components, especially collagen, due to an imbalance between the amount of matrix macromolecules produced versus degraded in the liver (NIH Guide PA-99-110 National Institute on Alcohol Abuse and Alcoholism). Historically, liver fibrosis has been considered to be an irreversible process involving the progressive replacement of the hepatic parenchyma with collagen-rich ECM (Muddu et al., Int J Biochem Cell Biol. 2006 Oct 7). Biochemical evidence for an increased deposition of collagen in humans has been demonstrated in patients with both acute and chronic forms of pulmonary fibrosis (Laurent, Ciba Found Symp. 1985 114:222-33). In idiopathic pulmonary fibrosis, there is an exorbitant production of extracellular matrix molecules, including collagen, tenascin, and proteoglycans (Noble et al., Clin Chest Med 2004 25:749-758). The mechanisms which control the step from fibrogenesis (a potentially reversible reaction) to fibrosis (irreversible) may be linked to the maturation of collagen, calcification, or the formation of cross-linked protein masses (Toxicol Pathol. 1991;19(4 Pt 1):526-39.). As described herein and shown in the Examples, it has been found through RNA inhibition experiments that inhibition of p38, including but not limited to the p38γ isoform, can affect levels of collagen production in target cells, including fibroblasts.

RNA interference (RNAi) is a cellular mechanism in which short double stranded RNAs, known as short interfering RNAs or siRNAs, direct the degradation of a complementary RNA target. This process can lead to a significant reduction in the level of the RNA target. Elbashir and coworkers (2001 Nature 411: 494-8) defined the structural features of a typical siRNA as a 19 base pair duplex with two nucleotide overhangs on both ends. Researchers have subsequently used this format to design synthetic siRNAs in which one of the two RNA oligonucleotides is complementary to a gene of interest. The introduction of these synthetic siRNAs into mammalian cells can promote degradation of the complementary mRNA and a corresponding reduction in the corresponding protein product. The use of RNAi technology allows functional studies in which reduction of the expression of specific genes allows experiments to investigate the role of that gene in a cellular pathway or disease state.

Short hairpin RNAs (or shRNAs) are a related research tool in which the 21 nucleotide duplex described by Elbashir and coworkers is converted into a hairpin format. The hairpin design allows for the introduction of expression cassettes into cells that direct shRNA expression from an appropriate transcriptional promoter. The use of shRNAs eliminates the need for efficient transfection of cells with synthetic siRNAs, which can be difficult with certain cell types, and therefore expands the number of cell types that can be successfully used for RNAi-based experiments. The use of shRNAs also allows extended/stable knockdown of an mRNA target and the isolation of cell populations that have been transduced with an shRNA expression cassette.

One modality of the stable introduction of shRNA expression cassettes is attenuated lentiviruses. Moffat and coworkers have described a library of attenuated lentiviral particles that are designed to stably incorporate a U6-promoter driven shRNA expression cassette into the genome of infected cells (Moffat et al 2006 Cell 124: 1283-98). The transferred segment of the engineered virus also encodes a puromycin resistance marker that can be used to select cells that have a stably integrated shRNA expression cassette.

When eight lentiviral particles containing coding regions (SEQ ID NOS:1-8) encoding different shRNAs targeting p38α or p38γ were prepared and introduced into cells as described in Example 10, mRNAs isolated and quantitated according to Example 11, and collagen levels assayed according to Example 12, the results showed that sequences that decrease p38γ mRNA levels were associated with resulting decrease in accumulation of collagen. See Table 1 in Example 12. Thus, inhibition of, for example, p38γ, can result in decreased levels of collagen expression, and, therefore, can inhibit fibrosis.

### p38 and apoptosis

It appears that concomitant activation of p38 and apoptosis is induced by a variety of agents such as NGF withdrawal and Fas ligation. Cysteine proteases (caspases) are central to the apoptotic pathway and are expressed as inactive zymogens. Caspase inhibitors may then block p38 activation through Fas cross-linking. However, overexpression of dominant active MKK6b can also induce caspase activity and cell death. The role of p38 in apoptosis is cell type- and stimulus-dependent. While p38 signaling has been shown to promote cell death in some cell lines, in different cell lines p38 has been shown to enhance survival, cell growth, and differentiation.

### p38 in the cell cycle

Overexpression of p38α in yeast leads to significant slowing of proliferation, indicating involvement of p38α in cell growth. A slower proliferation of cultured mammalian cells was observed when the cells were treated with p38α/β inhibitor, SB203580.

### p38 and cardiomyocyte hypertrophy

Activation and function of p38 in cardiomyocyte hypertrophy has been studied. During progression of hypertrophy, both p38α and p38β levels were increased and constitutively active MKK3 and MKK6-elicited hypertrophic responses enhanced by sarcomeric organization and elevated atrial natriuretic factor expression. Also, reduced signaling of p38 in the heart promotes myocyte differentiation via a mechanism involving calcineurin-NFAT signaling.

### p38 and development

Despite the non-viability of p38 knockout mice, evidence exists regarding the differential role of p38 in development. p38 has been linked to placental angiogenesis but not cardiovascular development in several studies. Furthermore, p38 has also been linked to erythropoietin expression suggesting a role in erythropoiesis. PRAK has recently been implicated in cell development in murine implantation. PRAK mRNA, as well as p38 isoforms, were found to be expressed throughout blastocyst development

### p38 and cell differentiation

p38α and/or p38β were found to play an important role in cell differentiation for several different cell types. The differentiation of 3T3-L1 cells into adipocytes and the differentiation of PC12 cells into neurons both require p38α and/or β. The p38 pathway was found to be necessary and sufficient for SKT6 differentiation into hemoglobinized cells as well as C2C112 differentiation in myotubules.

### p38 in senescence and tumor suppression

p38 has a role in tumorigenesis and senescence. There have been reports that activation of MKK6 and MKK3 led to a senescent phenotype dependent upon p38 MAPK activity. Also, p38 MAPK activity was shown responsible for senescence in response to telomere shortening, H₂O₂ exposure, and chronic RAS oncogene signaling. A common feature of tumor cells is a loss of senescence and p38 is linked to tumorigenesis in certain cells. It has been reported that p38 activation is reduced in tumors and that loss of components of the p38 pathway such as MKK3 and MKK6 resulted in increased proliferation and likelihood of tumorigenic conversion regardless of the cell line or the tumor induction agent used in these studies.

### p38 MAP kinase inhibitors

A "p38 MAPK inhibitor" is a compound that inhibits the activity of p38. The inhibitory effects of a compound on the activity of p38 may be measured by various methods well-known to a skilled artisan. For example, the inhibitory effects may be measured by measuring the level of inhibition of lipopolysaccharide (LPS)-stimulated cytokine production (Lee et al. 1988 Int J Immunopharmacol 10:835-843; Lee et al. 1993 Ann NY Acad Sci 696:149-170; Lee et al. 1994 Nature 372:739-746; Lee et al. 1999 Pharmacol Ther 82:389-397).

Efforts to develop p38 MAPK inhibitors have generally focused on increasing potency. SB203580 was found to be a potent p38 kinase inhibitor with an IC₅₀ valus in nanomolar range. For example, for SB203580 the IC₅₀ was found to be 48 nM. The pyridinylimidazoles SKF 86002 (**P1**) and SB203582 (**P2**) shown below are also p38 kinase inhibitors. Recent publications (Lee et al. 2000 Immunopharmacology 47:185-201) have disclosed the p38 inhibitors **(P3- P6)** shown below. Notable among these inhibitors is the relatively high potency and selectivity described for compound **P4** (p38 IC₅₀ = 0.19 nM) and the inhibition of inflammation driven angiogenesis by SB 220025 (P6). Such compounds are believed to bind in the ATP pocket of the p38α polypeptide, and are competitive inhibitors of the cofactor ATP.

Certain pyrimidine imidazoles were also found to be potent p38 kinase inhibitors with subnanomolar IC50 values (Liverton et al. 1999 J. Med. Chem. 42: 2180-2190). Such compounds are believed to bind in the ATP pocket of the p38α polypeptide, and are competitive inhibitors of the essential cofactor ATP.

Certain diaryl ureas, for example BIRB 796, are also potent inhibitors of p38 MAPK (Pargellis et al. Nature Structural Biology 9:268-272). BIRB 796 has an IC₅₀ against TNFα of 18 nM. The diaryl urea is structurally distinct from the pyridinyl imidazoles and pyrimidinyl imidazole inhibitors of p38. Certain diaryl ureas are thought to be allosteric inhibitors of p38α. It is believed that upon binding p38α at a site spatially removed from the ATP binding pocket, the allosteric inhibitor induces a conformational change in the ATP binding pocket of the polypeptide and the conformational change results in an ATP binding pocket incompatible with efficient binding of ATP, thus accounting for the observed potency of the compound as a p38α inhibitor.

Two p38 inhibitors reported to be in clinical development are HEP689 (P7) and VX-745 **(P8).** VX-745 is reportedly in Phase II trials for rheumatoid arthritis. Potent topical anti-inflammatory activity has been disclosed for HRP689, which has reportedly entered clinical development to explore its potential as a topical agent for the treatment of psoriasis and other skin disorders.

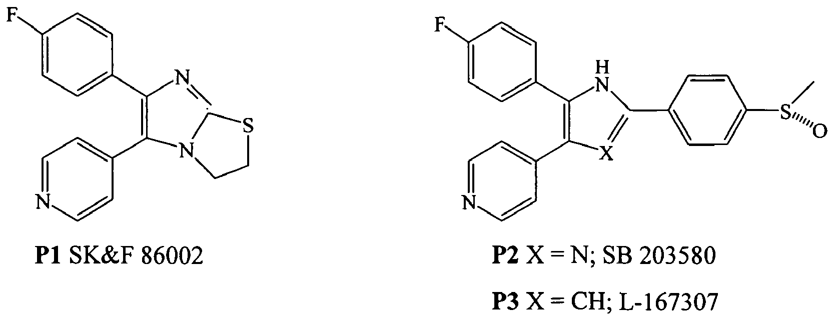

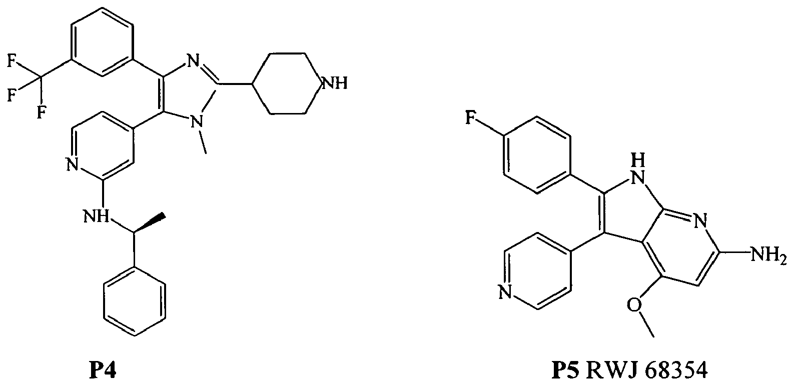

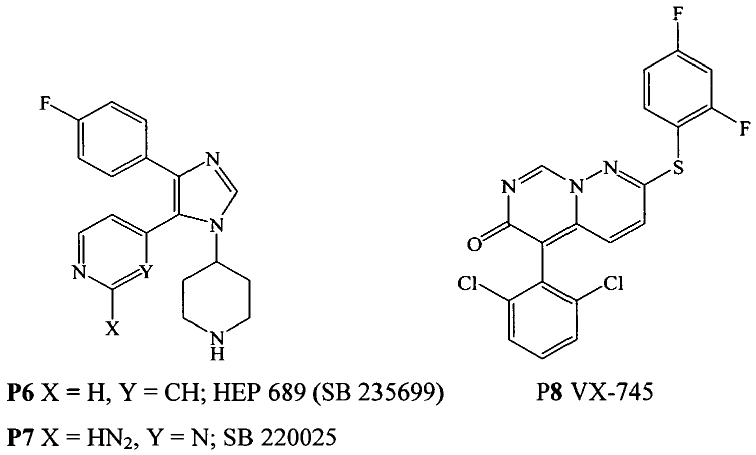

Further discussion of various p38 inhibitors can be found in Boehm et al. 2000 Exp Opin Ther Pat 10:25-37; and Salituro et al. 1999 Curr Med Chem 6:807-823 and Fitzgerald et al. 2003 Nature Structural Biology 10:764-769.

It has been discovered that the γ isoform of p38 bears significant structural similarity to the α isoform. However, while important residues are conserved between the isoforms, structural variation is observed including in the region around the ATP binding pocket of the protein. Modeling of the p38 inhibitors described above demonstrated that energetically unfavorable interactions were observed when the p38α inhibitors interacted with the p38γ isoform. Modeling also revealed that other, energetically favorable interactions were retained when the p38α inhibitors interacted with the p38γ isoform.

It has been found that certain compounds of Genus II lack sterically unfavorable interactions, for example, the sterically unfavorable interaction between Met109 of p38γ and the trifluoromethylphenyl moiety of compound 1 described in Fitzgerald et al. 2003 Nature Structural Biology 10:764-769, and further that certain compounds of Genus II have substituents which interact with p38γ and impart positive binding energy. It has been found that simple removal of the trifluoromethylphenyl moiety may lack the favorable binding energy of a compound bearing a substituent interacting with the protein. Accordingly, in some embodiments, the trifluoromethylphenyl substituent in compound 1 of Fitzgerald et al. 2003 Nature Structural Biology 10:764-769 is replaced by substituents of smaller molecular volume, or by substituents of smaller molecular volume, and which make van der Waals interactions with the hydrophobic pocket defined by the p38γ residues Leu89, Leu58, Leu107 and Leu108, or by substituents of smaller molecular volume and which make hydrogen-bonding or electrostatic interaction with Met109 of p38γ, or by substituents of smaller molecular volume and which make van der Walls interactions with the hydrophobic pocket defined by the p38γ residues Leu89, Leu58, Leu107 and Leu108, and which also make hydrogen-bonding or electrostatic interaction with Met 109 of p38γ.

It has been found that certain compounds of Genus IV lack sterically unfavorable interactions, for example the sterically unfavorable interaction between Met109 of p38γ and the fluorophenyl moiety of **P2**, and further that certain compounds of Genus IV have substituents which interact with p38γ and impart positive binding energy. Accordingly, in some embodiments, the fluorophenyl substituent of **P2** is replaced by substituents of smaller molecular volume, or by substituents of smaller molecular volume and which make van der Waals interactions with the hydrophobic pocket defined by the p38γ residues Leu89, Leu58, Leu107 and Leu108, or by substituents of smaller molecular volume and which make hydrogen-bonding or electrostatic interaction with Met109 of p38γ, or by substituents of smaller molecular volume and which make van der Waals interactions with the hydrophobic pocket defined by the p38γ residues Leu89, Leu58, Leu107 and Leu108, and which also make hydrogen-bonding or electrostatic interaction with Met 109 of p38γ.

It has been found that certain compounds of Genus III lack sterically unfavorable interactions, for example the sterically unfavorable interaction between Met109 of p38γ and the substituted naphthylene moiety of BIRB 796, and further that certain compounds of Genus III have substituents which interact with p38γ and impart positive binding energy. Accordingly, in some embodiments, the substituted naphthylene of BIRB 796 is replaced by substituents of smaller molecular volume, or by substituents of smaller molecular volume and which make van der Waals interactions with the hydrophobic pocket defined by the p38γ residues Leu89, Leu58, Leu107 and Leu108, or by substituents of smaller molecular volume and which make hydrogen-bonding or electrostatic interaction with Met109 of p38γ, or by substituents of smaller molecular volume which make π-stacking or π-cation interactions with Phe111 of p38γ, or by substituents of smaller molecular volume and which make van der Walls interactions with the hydrophobic pocket defined by the p38γ residues Leu89, Leu58, Leu107 and Leu108, and which also make hydrogen-bonding or electrostatic interaction with Met109 of p38γ, or by substituents of smaller molecular volume and which make van der Walls interactions with the hydrophobic pocket defined by the p38γ residues Leu89, Leu58, Leu107 and Leu108, and which also make π-stacking or π-cation interactions with Phe111 I of p38γ, or by substituents of smaller molecular volume and which make van der Walls interactions with the hydrophobic pocket defined by the p38γ residues Leu89, Leu58, Leu107 and Leu108, and which make hydrogen-bonding or electrostatic interaction with Met109 of p38γ and which also make π-stacking or π-cation interactions with Phe111 of p38γ.

Preferred p38 inhibitors described herein are bicyclic oxopyridine derivatives and analogs that exhibit relatively high potency of p38γ inhibition and having a relatively high therapeutic effect (e.g., for modulating an SAPK system) as a result of such inhibition. Preferably, the p38 inhibitors of the embodiments exhibit an IC₅₀ in the range of about 10 pM to about 500 µM, preferably about 10 pM to about 5 µM or about 100 nM to about 500 µM, preferably about 50 pM to about 1 µM, more preferably about 50 pM to about 200 nM for the inhibition of the p38γ MAPK. Preferably, the p38 inhibitors of the embodiments exhibit an IC₅₀ value for the inhibition of the p38α which is at least about twofold higher than the IC_{50 value} for the inhibition of the p38γ MAPK. More preferably, the p38 inhibitors of the embodiments exhibit an IC₅₀ value for the inhibition of the p38α which is at least about fivefold higher than the IC₅₀ value for the inhibition of the p38γ MAPK. Even more preferably, the p38 inhibitors of the embodiments exhibit an IC₅₀ value for the inhibition of the p38α which is at least about tenfold higher than the IC₅₀ value for the inhibition of the p38γ MAPK.

Preferred p38 inhibitors described herein are pyrimidinyl imidazole derivatives and analogs that exhibit relatively high potency of p38γ inhibition and having a relatively high therapeutic effect (e.g., for modulating an SAPK system) as a result of such inhibition. Preferably, the p38 inhibitors of the embodiments exhibit an IC₅₀ in the range of about 10 pM to about 500 µM, preferably about 10 pM to about 5 µM or about 100 nM to about 500 µM, preferably about 50 pM to about 1 µM more preferably about 50 pM to about 200 nM for the inhibition of the p38γ MAPK. Preferably, the p38 inhibitors of the embodiments exhibit an IC₅₀ value for the inhibition of the p38α which is about twofold higher than the IC₅₀ value for the inhibition of the p38γ MAPK. More preferably, the p38 inhibitors of the embodiments exhibit an IC₅₀ value for the inhibition of the p38α which is at least about fivefold higher than the IC₅₀ value for the inhibition of the p38γ MAPK. Even more preferably, the p38 inhibitors of the embodiments exhibit an IC₅₀ value for the inhibition of the p38α which is at least about tenfold higher than the IC₅₀ value for the inhibition of the p38γ MAPK

Preferred p38 inhibitors described herein are diaryl urea derivatives and analogs that exhibit relatively high potency of p38γ inhibition and having a relatively high therapeutic effect (e.g., for modulating an SAPK system) as a result of such inhibition. Preferably, the p38 inhibitors of the embodiments exhibit an IC₅₀ in the range of about 10 pM to about 500 µM, preferably about 10 pM to about 5 µM or about 100 nM to about 500 µM, preferably about 50 pM to about 1 µM more preferably about 50 pM to about 200 nM for the inhibition of the p38γ MAPK. Preferably, the p38 inhibitors of the embodiments exhibit an IC₅₀ value for the inhibition of the p38α which is about twofold higher than the IC₅₀ value for the inhibition of the p38γ MAPK.

The term "alkyl" used herein refers to a monovalent straight or branched chain radical of from one to ten carbon atoms, including, but not limited to, methyl, ethyl, n-propyl, isopropyl, n-butyl, isobutyl, tert-butyl, n-hexyl, and the like.

The term "alkenyl" used herein refers to a monovalent straight or branched chain radical of from two to ten carbon atoms containing a carbon double bond including, but not limited to, 1-propenyl, 2-propenyl, 2-methyl-1-propenyl, 1-butenyl, 2-butenyl, and the like.

The term "halo" used herein refers to fluoro, chloro, bromo, or iodo.

The term "haloalkyl" used herein refers to one or more halo groups appended to an alkyl radical.

The term "nitroalkyl" used herein refers to one or more nitro groups appended to an alkyl radical.

The term "thioalkyl" used herein refers to one or more thio groups appended to an alkyl radical.

The term "hydroxyalkyl" used herein refers to one or more hydroxy groups appended to an alkyl radical.

The term "alkoxy" used herein refers to straight or branched chain alkyl radical covalently bonded to the parent molecule through an --O-- linkage. Examples of alkoxy groups include, but are limited to, methoxy, ethoxy, propoxy, isopropoxy, butoxy, n-butoxy, sec-butoxy, t-butoxy and the like.

The term "alkoxyalkyl" used herein refers to one or more alkoxy groups appended to an alkyl radical.

The term "carboxy" used herein refers to -COOH.

The term "alkoxycarbonyl" refers to -(CO)-O-alkyl. Examples of alkoxycarbonyl groups include, but are limited to, methoxycarbonyl group, ethoxycarbonyl group, propoxycarbonyl group, and the like.

As used herein, a radical indicates species with a single, unpaired electron such that the species containing the radical can be covalently bonded to another species. Hence, in this context, a radical is not necessarily a free radical. Rather, a radical indicates a specific portion of a larger molecule. The term "radical" can be used interchangeably with the term "group."

As used herein, a substituted group is derived from the unsubstituted parent structure in which there has been an exchange of one or more hydrogen atoms for another atom or group. When substituted, the substituent group(s) is (are) one or more group(s) individually and independently selected from alkyl, cycloalkyl, aryl, fused aryl, heterocyclyl, heteroaryl, hydroxy, alkoxy, aryloxy, mercapto, alkylthio, arylthio, cyano, halo, carbonyl, thiocarbonyl, alkoxycarbonyl, nitro, silyl, trihalomethanesulfonyl, trifluoromethyl, and amino, including mono- and di-substituted amino groups, and the protected derivatives thereof. The protecting groups that can form the protective derivatives of the above substituents are known to those of skill in the art and can be found in references such as Greene and Wuts Protective Groups in Organic Synthesis; John Wiley and Sons: New York, 1999. Wherever a substituent is described as "optionally substituted" that substituent can be substituted with the above substituents.

The term "purified" refers to a compound which has been separated from other compounds such that it comprises at least 95% of the measured substance when assayed.

Asymmetric carbon atoms may be present in the compounds described herein. All such isomers, including diastereomers and enantiomers, as well as the mixtures thereof are intended to be included in the scope of the recited compound. In certain cases, compounds can exist in tautomeric forms. All tautomeric forms are intended to be included in the scope of the recited compound. Likewise, when compounds contain an alkenyl or alkenylene group, there exists the possibility of cis- and trans- isomeric forms of the compounds. Both cis- and trans- isomers, as well as the mixtures of cis- and trans- isomers, are contemplated. Thus, reference herein to a compound includes all of the aforementioned isomeric forms unless the context clearly dictates otherwise.

Various forms are included in the embodiments, including polymorphs, solvates, hydrates, conformers, salts, and prodrug derivatives. A polymorph is a composition having the same chemical formula, but a different structure. A solvate is a composition formed by solvation (the combination of solvent molecules with molecules or ions of the solute). A hydrate is a compound formed by an incorporation of water. A conformer is a structure that is a conformational isomer. Conformational isomerism is the phenomenon of molecules with the same structural formula but different conformations (conformers) of atoms about a rotating bond. Salts of compounds can be prepared by methods known to those skilled in the art. For example, salts of compounds can be prepared by reacting the appropriate base or acid with a stoichiometric equivalent of the compound. A prodrug is a compound that undergoes biotransformation (chemical conversion) before exhibiting its pharmacological effects. For example, a prodrug can thus be viewed as a drug containing specialized protective groups used in a transient manner to alter or to eliminate undesirable properties in the parent molecule. Thus, reference herein to a compound includes all of the aforementioned forms unless the context clearly dictates otherwise.

The compounds described below are useful in the methods described herein. In an embodiment, a compound as described below exhibits an IC₅₀ in the range of about 10 pM to about 5 µM for inhibition of p38γ, preferably about 100 nm to about 500 µM.

An embodiment provides a family of compounds represented by the following genus (Genus I):

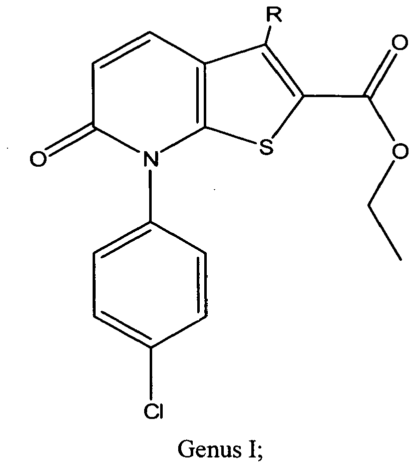

wherein R is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.

Another embodiment provides a family of compounds represented by the following genus (Genus ID:

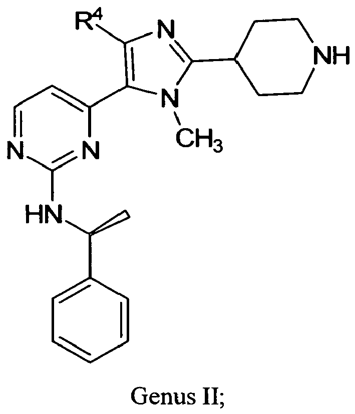

wherein R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.

Another embodiment provides a family of compounds represented by the following genus (Genus III):

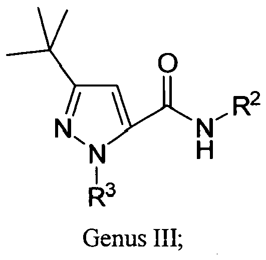

wherein

R² and R³ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.

In one embodiment, the compound of Genus III is not 1-(5-tert-Butyl-2-p-tolyl-2H-pyrazol-3-yl)-3-[4-(2-morpholin-4-yl-ethoxy)naph- thalen-1-yl]urea (BIRB 796).

Another embodiment provides a family of compounds represented by the following Genus (Genus IV):

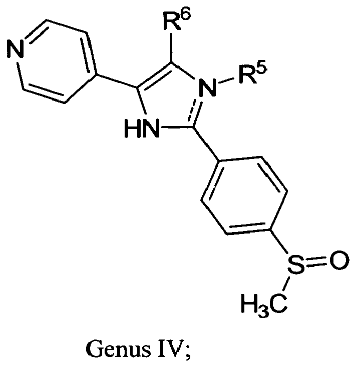

wherein

R⁵ and R⁶ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.

In one embodiment, the compound of Genus IV is not 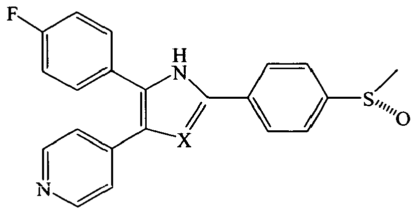 where X is N.

It will be recognized that a particular compound described herein may be a member of more than one of the various genera described above. The compounds described herein are useful for modulating a stress activated protein kinase (SAPK) system.

Another embodiment is directed to purified compounds represented by Genera I-IV. The degree of purity may be expressed as a percentage as described above. In preferred embodiments, purified compounds represented by Genera I-IV, have a purity of about 96% or greater, more preferably about 98% or greater, by weight based on total weight of the composition that comprises the purified compound.

Compounds of Genera I-IV may be synthesized by using various conventional reactions known in the art. Examples of syntheses include the synthetic schemes presented in Examples 6, 7, 8 and 9.

As bicyclic oxopyridine derivatives, compounds of Genus I may also be synthesized by any conventional reactions known in the art based on the known synthetic schemes for bicyclic oxopyridines, such as given in Example 6.

As pyrimidinyl imidiazole derivatives, compounds of Genera II and IV may also be synthesized by any conventional reactions known in the art, including those based on known synthetic schemes for pyrimidinyl imidazoles, e.g., as illustrated in Examples 7 and 9.

As diaryl urea derivatives, compounds of Genus III may also be synthesized by any conventional reactions known in the art, including those based on known synthetic schemes for diaryl ureas, e.g., as illustrated in Example 8.

Starting materials described herein are available commercially, are known, or can be prepared by methods known in the art. Additionally, starting materials not described herein are available commercially, are known, or can be prepared by methods known in the art.

Starting materials can have the appropriate substituents to ultimately give desired products with the corresponding substituents. Alternatively, substituents can be added at any point of synthesis to ultimately give desired products with the corresponding substituents.

The synthetic scheme depicted in Example 6 shows methods that may be used to prepare the compounds of Genus I. The synthetic schemes depicted in Examples 7 and 9 show methods that may be used to prepare the compounds of Genera II and IV. The synthetic scheme depicted in Example 8 shows methods that may be used to prepare the compounds of Genus III. One skilled in the art will appreciate that a number of different synthetic reaction schemes may be used to synthesize the compounds of Genera I-IV. Further, one skilled in the art will understand that a number of different solvents, coupling agents, and reaction conditions can be used in the syntheses reactions to yield comparable results.

One skilled in the art will appreciate variations in the sequence and, further, will recognize variations in the appropriate reaction conditions from the analogous reactions shown or otherwise known which may be appropriately used in the processes above to make the compounds of Genera I-IV.

In the processes described herein for the preparation of the compounds of compounds of Genera I-IV, the use of protective groups is generally well recognized by one skilled in the art of organic chemistry, and accordingly the use of appropriate protecting groups may in some cases be implied by the processes of the schemes herein, although such groups may not be expressly illustrated. Introduction and removal of such suitable protecting groups are well known in the art of organic chemistry; see for example, T.W. Greene, "Protective Groups in Organic Synthesis", Wiley (New York), 1999. The products of the reactions described herein may be isolated by conventional means such as extraction, distillation, chromatography, and the like.

The salts, e.g., pharmaceutically acceptable salts, of the compounds of Genera I-IV may be prepared by reacting the appropriate base or acid with a stoichiometric equivalent of the compounds. Similarly, pharmaceutically acceptable derivatives (e.g., esters), metabolites, hydrates, solvates and prodrugs of the compounds of Genera I-IV may be prepared by methods generally known to those skilled in the art. Thus, another embodiment provides compounds that are prodrugs of an active compound. In general, a prodrug is a compound which is metabolized *in vivo* (e.g., by a metabolic transformation such as deamination, dealkylation, de-esterification, and the like) to provide an active compound. A "pharmaceutically acceptable prodrug" means a compound which is, within the scope of sound medical judgment, suitable for pharmaceutical use in a patient without undue toxicity, irritation, allergic response, and the like, and effective for the intended use, including a pharmaceutically acceptable ester as well as a zwitterionic form, where possible, of the compounds of the embodiments. Examples of pharmaceutically-acceptable prodrug types are described in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The compounds and compositions described herein may also include metabolites. As used herein, the term "metabolite" means a product of metabolism of a compound of the embodiments or a pharmaceutically acceptable salt, analog, or derivative thereof, that exhibits a similar activity in vitro or in vivo to a compound of the embodiments. The compounds and compositions described herein may also include hydrates and solvates. As used herein, the term "solvate" refers to a complex formed by a solute (herein, a compound of Genera I-IV) and a solvent. Such solvents for the purpose of the embodiments preferably should not interfere with the biological activity of the solute. Solvents may be, by way of example, water, ethanol, or acetic acid. In view of the foregoing, reference herein to a particular compound or genus of compounds will be understood to include the various forms described above, including pharmaceutically acceptable salts, esters, prodrugs, metabolites and solvates thereof.

### Screening a library of compounds for p38y preferential inhibitors

In another aspect, a method is provided for identifying a pharmaceutically active compound, e.g., for determining whether a compound is potentially useful as a therapeutic agent, e.g., for the prevention or treatment of a fibrotic condition (such as p38- or cytokine-associated condition). The method includes assaying a plurality of compounds for inhibition of a p38γ, selecting a compound which exhibits a relatively high potency for inhibiting a p38γ, and further testing the selected compound for inhibition of a p38α and selecting a compound which also exhibits a relatively high potency for inhibiting a p38α. The method also includes assaying a plurality of compounds for inhibition of a p38γ, selecting a compound which exhibits a relatively high potency for inhibiting a p38γ and which also exhibits a relatively high potency for inhibiting a p38α. The method includes assaying a plurality of compounds for inhibition of a p38α, selecting a compound which exhibits a relatively high potency for inhibiting a p38α and which also exhibits a relatively high potency for inhibiting a p38γ. Preferably, an IC₅₀ of such a high-potency p38γ and p38α inhibitor compound is in the range of about 10 pM and about 500 µM, preferably about 10 pM to about 5 µM or about 100 nM to about 500 µM, more preferably about 500 pM to about 1 µM, even more preferably about 500 pM to about 1 µM for inhibition of the p38γ. Preferably, an IC₅₀ value against p38α of such a high-potency p38γ and p38α inhibitor compound is greater than twice its IC₅₀ value against p38γ. More preferably, an IC₅₀ value against p38α of such a high-potency p38γ and p38α inhibitor compound is greater than fivefold its IC₅₀ value against p38γ. Even more preferably, an IC₅₀ value against p38α of such a high-potency p38γ and p38α inhibitor compound is greater than tenfold its IC₅₀ value against p38γ. The plurality of compounds to be assayed is preferably selected from a library of potential compounds. The assaying of the plurality of compounds from the library may be conducted in various ways. For example, in some embodiments, the methods further comprise contacting a p38 MAPK with the plurality of compounds, and determining whether the compounds inhibit the activity of cytokines. The p38 MAPK is preferably selected from the group consisting of p38α and p38γ. In preferred embodiments, the contacting step takes place *in vitro;* in certain preferred embodiments, the contacting step comprises contacting a cell comprising p38 MAPK with the compound.

In yet another embodiment, methods are provided for inhibiting the activity of p38 MAPK in a cell, *in vitro* or *in vivo.* In general, such methods include contacting a cell containing a p38 MAPK with an effective p38-inhibiting amount of a compound (e.g., a compound of Genera I-IV), under conditions such that p38 activity in the cell is inhibited. Examples of such methods are provided in the EXAMPLES section below. The compound preferably exhibits an IC₅₀ in the range of about 10 pM and about 500 µM, preferably about 10 pM to about 5 µM or about 100 nM to about 500 µM, more preferably about 500 pM to about 1 µM, even more preferably about 500 pM to about 1 µM for inhibition of the p38γ. Preferably, an IC₅₀ value against p38α of the compound is greater than twice its IC₅₀ value against p38γ.

*In vivo* methods include for example, introducing into a group of animals orally or by injection a compound of interest (e.g., a compound of Genera I-IV) in various concentrations. Following the introduction of the compound, lipopolysaccharide is administered intravenously. Serum TNFα levels are measured and compared to that from control animals. The preferred compounds inhibit the release of TNFα, thus reducing TNFα levels in the blood samples of the tested animals. The compound preferably exhibits an EC₅₀ in the range of about 10 pM to about 5 µM, more preferably about 500 pM to about 1 µM, even more preferably about 500 pM to about 1 µM for inhibition of the release of TNFα.

The method of identifying a pharmaceutically active compound may further include determining a mammalian toxicity of the selected compound. Such methods are generally known to those skilled in the art. The method of identifying a pharmaceutically active compound may also include administering the selected compound to a test subject, either in conjunction with the determination of mammalian toxicity or for other reasons. In an embodiment, the test subject test subject has or is at risk for having an inflammatory condition. Preferably the test subject is a mammal, and may be a human.

### Methods of inhibiting p38 MAP kinase

In an embodiment, methods are provided for modulating a SAPK system, *in vitro* or *in vivo.* The methods include contacting a SAPK-modulating concentration of a compound with a p38 MAPK (e.g., by contacting the compound with a cell or tissue containing the p38 MAPK), where the compound has a relatively high potency for inhibition of the p38γ, corresponding to a relatively high inhibitory concentration for inhibition of the p38 MAPK by the compound.

The inhibitory concentration (IC) is a concentration that results in a reduction in the activity of p38 MAPK by a specified percentage (e.g., 50%, 40%, 30%, 20%, 10%) on a dose-response curve. For example, IC₅₀, IC₄₀, IC₃₀, IC₂₀ and IC₁₀ are determined as concentrations that result in reductions in the activity of p38 MAPK by 50%, 40%, 30%, 20% and 10%, respectively on a dose-response curve. The IC₅₀ of the SAPK system-modulating compound is preferably in the range of about 10 pM and about 500 µM, preferably about 10 pM to about 5 µM or about 100 nM to about 500 µM, more preferably about 50 pM to about 1 µM, even more preferably about 50 pM to about 1 µM for inhibition of the p38γ.

"Contacting a cell" refers to a condition in which a compound or other composition of matter is in direct contact with a cell or tissue, or is close enough to induce a desired biological effect in a cell or tissue. For example, contacting a cell or tissue containing p38 MAPK with a compound may be conducted in any manner that permits an interaction between p38 MAPK and the compound, resulting in the desired biological effect in a cell. Contacting a cell or tissue may be accomplished, for example, by intermixing or administration of a compound (such as a compound of Genera I-IV; and/or a salt, ester, prodrug and/or intermediate thereof, and/or a pharmaceutical composition comprising one or more of the foregoing).

Alternatively, contacting a cell or tissue may be accomplished by introducing a compound in a manner such that the compound will be targeted, directly or indirectly, to a cell or tissue containing p38 MAPK. Contacting a cell or tissue may be accomplished under conditions such that a compound binds to the p38 MAPK. Such conditions may include proximity of the compound and p38-containing cell or tissue, pH, temperature, or any condition that affects the binding of a compound to p38 MAPK.

In certain embodiments, the cell is contacted with the compound *in vitro;* in other embodiments, the cell is contacted with the compound *in vivo.*

When the cell is contacted *in vivo,* the effective concentration (EC) is a concentration that results in a reduction in the activity of p38 MAPK by a specified percentage (e.g., 50%, 40%, 30%, 20%, 10%) as measured by a specific physiological response which depends on the reduction of the activity of p38 MAPK. Such physiological response may be, for example, reduction in blood or other bodily fluid concentration of TNFα. For example, EC₅₀, EC₄₀, EC₃₀, EC₂₀ and EC₁₀ are determined as concentrations that result in reductions in the activity of p38 MAPK as measured by reduction in TNFα concentration by 50%, 40%, 30%, 20% and 10%, respectively on a dose-response curve. The EC₅₀ of the SAPK system-modulating compound is preferably in the range of about 10 pM and about 500 µM, preferably about 10 pM to about 5 µM or about 100 nM to about 500 µM, more preferably about 500 pM to about 1 µM, even more preferably about 500 pM to about 1 µM for inhibition of a p38γ.

In certain embodiments, the compound is provided in the form of a pharmaceutical composition, together with a pharmaceutically acceptable carrier.

### Methods of treatment and/or prevention

Another embodiment provides methods for treating or preventing disease states, e.g., fibrotic condition(s). The methods include identifying a subject at risk for or having an fibrotic condition and administering a compound to the subject in an effective amount to treat or prevent the fibrotic condition. In preferred embodiments, the compound exhibits an IC₅₀ in the range of about 10 pM and about 500 µM, preferably about 10 pM to about 5 µM or about 100 nM to about 500 µM, more preferably about 500 pM to about 1 µM, even more preferably about 500 pM to about 1 µM for inhibition of p38γ. In preferred embodiments, an IC₅₀ value against p38α of the compound is greater than twice its IC₅₀ value against p38γ. In preferred embodiments, the effective amount produces a blood or serum or another bodily fluid concentration that is equal to or less than an IC₅₀, or an IC₄₀, or an IC₃₀, or an IC₂₀, or an IC₁₀ for inhibition of p38γ by the compound and furthermore the effective amount produces a blood or serum or another bodily fluid concentration that is equal to or less than an IC₅₀, or an IC₄₀, or an IC30, or an IC₂₀, or an IC₁₀ for inhibition of p38α by the compound. In preferred embodiments, the compound exhibits an EC₅₀ in the range of about 10 pM to about 5 µM, more preferably about 500 pM to about 1 µM, even more preferably about 500 pM to about 1 µM for inhibition of the TNFα secretion. In other preferred embodiments, the effective amount produces a blood or serum or another bodily fluid concentration that is equal to or less than an EC₅₀, or an EC₄₀, or an EC₃₀, or an EC₂₀, or an EC₁₀ for inhibition of LPS-stimulated TNFα release in a bodily fluid by the compound. The effective amount is preferably about 70% or less, more preferably less than about 50%, of an amount that causes an undesirable side effect in the subject, such as, but not limited to, drowsiness, gastrointestinal upset, and photosensitivity rash. The compound used for the treatment or prevention is preferably a compound of Genera I-IV.

Methods for identifying a subject at risk for or having a fibrotic condition are known to those skilled in the art. Examples of fibrotic conditions that may be treated or prevented by the methods described herein include p-38 associated conditions, e.g., conditions associated with altered cytokine activity, conditions associated with modulation of an SAPK system, autoimmune diseases, and diseases associated with fibrosis. The cytokine (or cytokines) is (are) preferably selected from the group consisting of, but not limited to, IL-1β, IL-6, IL-8, and TNFα. In an embodiment, the compound used to treat or prevent the inflammatory condition is compound that inhibits a kinase in the SAPK signaling pathway. Examples of preferred compounds include compound of Genera I-IV.

The term "p38-associated condition" means a disease or other deleterious condition in which the p38 MAP kinase signaling pathway is implicated, whether directly or indirectly. Examples of p38-associated conditions include conditions caused by IL-1β, TNFα, IL-6 or IL-8 dysregulation or overexpression resulting from sustained, prolonged, enhanced or elevated levels of p38 activity. Such conditions include, without limitation, inflammatory diseases, autoimmune diseases, fibrotic diseases, destructive bone disorders, proliferative disorders, infectious diseases, neurodegenerative diseases, allergies, reperfusion ischemia in stroke, heart attacks, angiogenic disorders, organ hypoxia, vascular hyperplasia, cardiac hypertrophy, thrombin-induced platelet aggregation, and conditions associated with the prostaglandin or cyclooxygenase pathways, e.g., conditions involving prostaglandin endoperoxide synthase. A p38-associated condition can include any condition associated with or mediated by an isoform of p38.

A "fibrotic condition," "fibroproliferative condition," "fibrotic disease," "fibroproliferative disease," "fibrotic disorder," and "fibroproliferative disorder" are used interchangeably to refer to a condition, disease or disorder that is characterized by dysregulated proliferation or activity of fibroblasts and/or pathologic or excessive accumulation of collagenous tissue. Typically, any such disease, disorder or condition is amenable to treatment by administration of a compound as described herein. Fibrotic disorders include, but are not limited to, pulmonary fibrosis, including idiopathic pulmonary fibrosis (IPF) and pulmonary fibrosis from a known etiology, liver fibrosis, and renal fibrosis. Other exemplary fibrotic conditions include musculoskeletal fibrosis, cardiac fibrosis, post-surgical adhesions, scleroderma, glaucoma, and skin lesions such as keloids.

The term "modulating SAPK system" means increasing or decreasing activity of the stress-activated protein kinase system activity, e.g., by inhibiting p38 activity, whether *in vitro* or *in vivo.* In certain embodiments, the SAPK system is modulated when p38 activity in a cell is inhibited by about 50%, by about 40%, by about 30%, by about 20%, or by about 10% compared to the p38 activity of an untreated control cell.

A condition associated with altered cytokine activity, as used herein, refers to a condition in which cytokine activity is altered compared to a non-diseased state. This includes, but is not limited to, conditions caused by IL-1β, TNFα, IL-6 or IL-8 overproduction or dysregulation resulting in sustained, prolonged, enhanced or elevated levels of cytokine activity, which may be associated with p38 activity. Such conditions include, without limitation, inflammatory diseases, autoimmune diseases, fibrotic diseases, destructive bone disorders, proliferative disorders, infectious diseases, neurodegenerative diseases, allergies, reperfusion/ischemia in stroke, heart attacks, angiogenic disorders, organ hypoxia, vascular hyperplasia, cardiac hypertrophy, thrombin-induced platelet aggregation, and conditions associated with the cyclooxygenase and lipoxygenase signaling pathways, such as prostaglandin endoperoxide synthase. A cytokine-associated condition can include any condition associated with or mediated by IL-1 (particularly IL-1β), TNFα, IL-6 or IL-8, or any other cytokine which can be regulated by p38. In preferred embodiments, the cytokine associated condition is a condition associated with TNFα.

The methods described herein may also be used to treat autoimmune diseases and diseases associated with acute and chronic inflammation. These diseases include, but are not limited to: chronic obstructive pulmonary disease (COPD), inflammatory pulmonary fibrosis (IPF), rheumatoid arthritis; rheumatoid spondylitis; osteoarthritis; gout, other arthritic conditions; sepsis; septic shock; endotoxic shock; gram-negative sepsis; toxic shock syndrome; myofacial pain syndrome (MPS); Shigellosis; asthma; adult respiratory distress syndrome; inflammatory bowel disease; Crohn's disease; psoriasis; eczema; ulcerative colitis; glomerular nephritis; scleroderma; chronic thyroiditis; Grave's disease; Ormond's disease; autoimmune gastritis; myasthenia gravis; autoimmune hemolytic anemia; autoimmune neutropenia; thrombocytopenia; pancreatic fibrosis; chronic active hepatitis including hepatic fibrosis; acute and chronic renal disease; renal fibrosis, irritable bowel syndrome; pyresis; restenosis; cerebral malaria; stroke and ischemic injury; neural trauma; Alzheimer's disease; Huntington's disease; Parkinson's disease; acute and chronic pain; allergies, including allergic rhinitis and allergic conjunctivitis; cardiac hypertrophy, chronic heart failure; acute coronary syndrome; cachexia; malaria; leprosy; leishmaniasis; Lyme disease; Reiter's syndrome; acute synoviitis; muscle degeneration, bursitis; tendonitis; tenosynoviitis; herniated, ruptured, or prolapsed intervertebral disk syndrome; osteopetrosis; thrombosis; silicosis; pulmonary sarcosis; bone resorption diseases, such as osteoporosis or multiple myeloma-related bone disorders; cancer, including but not limited to metastatic breast carcinoma, colorectal carcinoma, malignant melanoma, gastric cancer, and non-small cell lung cancer; graft-versus-host reaction; and auto-immune diseases, such as Multiple Sclerosis, lupus and fibromyalgia; AIDS and other viral diseases such as Herpes Zoster, Herpes Simplex I or II, influenza virus, Severe Acute Respiratory Syndrome (SARS) and cytomegalovirus; and diabetes mellitus. In addition, the methods of the embodiments can be used to treat proliferative disorders (including both benign and malignant hyperplasias), including acute myelogenous leukemia, chronic myelogenous leukemia, Kaposi's sarcoma, multiple myeloma, breast cancer, including metastatic breast carcinoma; colorectal carcinoma; bone metastases, and the like; pain disorders including neuromuscular pain, headache, cancer pain, dental pain, and arthritis pain; angiogenic disorders including solid tumor angiogenesis, ocular neovascularization, and infantile hemangioma; conditions associated with the cyclooxygenase and lipoxygenase signaling pathways, including conditions associated with prostaglandin endoperoxide synthase-2 (including edema, fever, analgesia, and pain); organ hypoxia; thrombin-induced platelet aggregation. In addition, the methods described herein may be useful for the treatment of protozoal diseases in animals, including mammals.

A subject may include one or more cells or tissues, or organisms. A preferred subject is a mammal. A mammal may include any mammal. As a non-limiting example, preferred mammals include cattle, pigs, sheep, goats, horses, camels, buffalo, cats, dogs, rats, mice, and humans. A highly preferred subject mammal is a human. The compound(s) may be administered to the subject via any drug delivery route known in the art. Specific exemplary administration routes include oral, ocular, rectal, buccal, topical, nasal, ophthalmic, subcutaneous, intramuscular, intravenous (bolus and infusion), intracerebral, transdermal, and pulmonary.

The term "effective amount" as used in the context of "therapeutically effective amount" and "prophylactically effective amount", as used herein, refers to an amount of a compound sufficient to treat, ameliorate, or prevent the identified disease or condition, or to exhibit a detectable therapeutic, prophylactic, or inhibitory effect. The effect can be detected by, for example, the assays disclosed in the following examples. The precise effective amount for a subject will depend upon the subject's body weight, size, and health; the nature and extent of the condition; and the therapeutic or combination of therapeutics selected for administration. Therapeutically and prophylactically effective amounts for a given situation can be determined by routine experimentation that is within the skill and judgment of the clinician. Preferably, the effective amount of the compound of the embodiments produces a blood or serum or another bodily fluid concentration that is less than an IC₅₀, IC₄₀, IC₃₀, IC₂₀ or IC₁₀ for inhibition of p38 MAP kinase. Preferably, the effective amount of the compound of the embodiments produces a blood or serum or another bodily fluid concentration that is effective to alter TNFα secretion from whole blood by 10%, 15%, 20%, 30%, 40% or 50%.

For any compound, the therapeutically or prophylactically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans.

Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, ED₅₀/LD₅₀. Pharmaceutical compositions that exhibit large therapeutic indices are preferred. However, the pharmaceutical compositions that exhibit narrow therapeutic indices are also within the scope of the embodiments. The data obtained from cell culture assays and animal studies may be used in formulating a range of dosage for human use. The dosage contained in such compositions is preferably within a range of circulating concentrations that include an ED₅₀ with little or no toxicity. The dosage may vary within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

It will be appreciated that treatment as described herein includes preventing a disease, ameliorating symptoms, slowing disease progression, reversing damage, or curing a disease.

In one aspect, treating a fibrotic condition results in an increase in average survival time of a population of treated subjects in comparison to a population of untreated subjects. Preferably, the average survival time is increased by more than about 30 days; more preferably, by more than about 60 days; more preferably, by more than about 90 days; and even more preferably by more than about 120 days. An increase in survival time of a population may be measured by any reproducible means. In a preferred aspect, an increase in average survival time of a population may be measured, for example, by calculating for a population the average length of survival following initiation of treatment with an active compound. In an another preferred aspect, an increase in average survival time of a population may also be measured, for example, by calculating for a population the average length of survival following completion of a first round of treatment with an active compound.

In another aspect, treating a fibrotic condition results in a decrease in the mortality rate of a population of treated subjects in comparison to a population of subjects receiving carrier alone. In another aspect, treating a fibrotic condition results in a decrease in the mortality rate of a population of treated subjects in comparison to an untreated population. In a further aspect, treating a fibrotic condition results a decrease in the mortality rate of a population of treated subjects in comparison to a population receiving monotherapy with a drug that is not a compound of the embodiments, or a pharmaceutically acceptable salt, metabolite, analog or derivative thereof. Preferably, the mortality rate is decreased by more than about 2%; more preferably, by more than about 5%; more preferably, by more than about 10%; and most preferably, by more than about 25%. In a preferred aspect, a decrease in the mortality rate of a population of treated subjects may be measured by any reproducible means. In another preferred aspect, a decrease in the mortality rate of a population may be measured, for example, by calculating for a population the average number of disease-related deaths per unit time following initiation of treatment with an active compound. In another preferred aspect, a decrease in the mortality rate of a population may also be measured, for example, by calculating for a population the average number of disease related deaths per unit time following completion of a first round of treatment with an active compound.

In another aspect, treating a fibrotic condition results in a decrease in growth rate of a tumor. Preferably, after treatment, tumor growth rate is reduced by at least about 5% relative to prior to treatment; more preferably, tumor growth rate is reduced by at least about 10%; more preferably, reduced by at least about 20%; more preferably, reduced by at least about 30%; more preferably, reduced by at least about 40%; more preferably, reduced by at least about 50%; even more preferably, reduced by at least 60%; and most preferably, reduced by at least about 75%. Tumor growth rate may be measured by any reproducible means of measurement. In a preferred aspect, tumor growth rate is measured according to a change in tumor diameter per unit time.

In another aspect, treating a fibrotic condition results in a reduction in the rate of cellular proliferation. Preferably, after treatment, the rate of cellular proliferation is reduced by at least about 5%; more preferably, by at least about 10%; more preferably, by at least about 20%; more preferably, by at least about 30%; more preferably, by at least about 40%; more preferably, by at least about 50%; even more preferably, by at least about 60%; and most preferably, by at least about 75%. The rate of cellular proliferation may be measured by any reproducible means of measurement. In a preferred aspect, the rate of cellular proliferation is measured, for example, by measuring the number of dividing cells in a tissue sample per unit time.

In another aspect, treating a fibrotic condition results in a reduction in the proportion of proliferating cells. Preferably, after treatment, the proportion of proliferating cells is reduced by at least about 5%; more preferably, by at least about 10%; more preferably, by at least about 20%; more preferably, by at least about 30%; more preferably, by at least about 40%; more preferably, by at least about 50%; even more preferably, by at least about 60%; and most preferably, by at least about 75%. The proportion of proliferating cells may be measured by any reproducible means of measurement. In a preferred aspect, the proportion of proliferating cells is measured, for example, by quantifying the number of dividing cells relative to the number of nondividing cells in a tissue sample. In another preferred aspect, the proportion of proliferating cells is equivalent to the mitotic index.

In another aspect, treating a fibrotic condition results in a decrease in size of an area or zone of cellular proliferation. Preferably, after treatment, size of an area or zone of cellular proliferation is reduced by at least 5% relative to its size prior to treatment; more preferably, reduced by at least about 10%; more preferably, reduced by at least about 20%; more preferably, reduced by at least about 30%; more preferably, reduced by at least about 40%; more preferably, reduced by at least about 50%; even more preferably, reduced by at least about 60%; and most preferably, reduced by at least about 75%. Size of an area or zone of cellular proliferation may be measured by any reproducible means of measurement. In a preferred aspect, size of an area or zone of cellular proliferation may be measured as a diameter or width of an area or zone of cellular proliferation.

The methods described herein may include identifying a subject in need of treatment. In a preferred embodiment, the methods include identifying a mammal in need of treatment. In a highly preferred embodiment, the methods include identifying a human in need of treatment. Identifying a subject in need of treatment may be accomplished by any means that indicates a subject who may benefit from treatment. For example, identifying a subject in need of treatment may occur by clinical diagnosis, laboratory testing, or any other means known to one of skill in the art, including any combination of means for identification.

As described elsewhere herein, the compounds described herein may be formulated in pharmaceutical compositions, if desired, and can be administered by any route that permits treatment of the disease or condition. A preferred route of administration is oral administration. Administration may take the form of single dose administration, or the compound of the embodiments can be administered over a period of time, either in divided doses or in a continuous-release formulation or administration method (e.g., a pump). However the compounds of the embodiments are administered to the subject, the amounts of compound administered and the route of administration chosen should be selected to permit efficacious treatment of the disease condition.

The methods of the embodiments also include the use of a compound or compounds as described herein together with one or more additional therapeutic agents for the treatment of disease conditions. Thus, for example, the combination of active ingredients may be: (1) co-formulated and administered or delivered simultaneously in a combined formulation; (2) delivered by alternation or in parallel as separate formulations; or (3) by any other combination therapy regimen known in the art. When delivered in alternation therapy, the methods described herein may comprise administering or delivering the active ingredients sequentially, e.g., in separate solution, emulsion, suspension, tablets, pills or capsules, or by different injections in separate syringes. In general, during alternation therapy, an effective dosage of each active ingredient is administered sequentially, i.e., serially, whereas in simultaneous therapy, effective dosages of two or more active ingredients are administered together. Various sequences of intermittent combination therapy may also be used.

Diagnostic tests are contemplated as part of the methods described herein. For example, a tissue biopsy sample may be taken from a subject suffering from a fibrotic condition, e.g., a p38-associated or cytokine-associated condition. The biopsy sample can be tested to determine the level of p38 activity (or cytokine levels) present in the sample; the sample can then be contacted with a selected compound of the embodiments, and the p38 activity (or cytokine levels) measured to determine whether the compound has a desired effect (e.g., inhibition of p38 or cytokine activity with an IC₅₀ in the range of about 10 pM and about 500 µM, preferably about 10 pM to about 5 µM or about 100 nM to about 500 µM, more preferably about 50 pM to about 1 µM, even more preferably about 50 pM to about 1 µM for inhibition of the p38γ, and with an IC₅₀ value for inhibition of p38α at least twice the IC₅₀ value for inhibition of p38γ). Such a test may be used to determine whether treatment with such a compound is likely to be effective in that subject. Alternatively, the sample may be contacted with a labeled compound (e.g., a fluorescently-labeled compound, or a radioactivity-labeled compound) and the sample then examined and the fluorescent or radioactive signal detected to determine the distribution of p38 in the tissue sample. Repeated biopsy samples taken during a course of treatment may also be used to study the efficacy of the treatment. Other diagnostic tests using the compounds described herein will be apparent to one of ordinary skill in the art in light of the teachings of this specification.

Thus, for example, an embodiment provides methods for determining the presence, location, and/or quantity of p38 protein in a cell or tissue sample. The methods include: a) contacting the cell or tissue sample with a compound of the embodiments under conditions such that the compound can bind to a p38 MAPK; and b) determining the presence, location, and/or quantity of the compound in the cell or tissue sample, thereby determining the presence, location, and/or quantity of the p38 MAPK in the cell or tissue sample. Determining the presence, location, and/or quantity of the compound in the cell or tissue sample may be conducted by any means that reveals the presence, location, and/or quantity, of the compound in the cell or tissue. For example, as described previously, radioactive or fluorescent labeling methods may be used. Additional methods of determining the presence, location, and/or quantity of the compound will be apparent to a skilled artisan.

Another embodiment provides methods for determining: (1) whether a compound will be a useful therapeutic agent for treatment of a subject suffering from an inflammatory condition, or (2) the severity of disease or (3) the course of disease during treatment with a disease-modifying agent. The methods include: a) obtaining a cell or tissue sample from the subject before, during and after termination of treatment with a compound as described herein or another disease-modifying agent; b) contacting the sample with the compound; and c) determining the amount of the compound that binds to the sample, wherein binding to p38 MAPK by the compound is related to the amount of p38 MAPK in the sample.

### Specific examples of diseases contemplated to be treated by the compounds and methods described herein

### COPD

Chronic obstructive pulmonary disease (COPD) is characterized by a chronic inflammatory process in the lung that includes (1) increased number of inflammatory cells (neutrophils, macrophages and SD8⁺ T cells) in the airways and parenchyma, (2) increased inflammatory cytokine and chemokine expression, and (3) increased number of proteases (elastases, cathepsins, and matrix metalloproteinases, MMPs). The production and action of many of potential mediators of airway inflammation are believed to be dependent on the stress-induced MAPK or p38 kinase cascade. Several reports support the association pf p38 kinase activation with as plethora of pulmonary events: LPS- and TNF-α-induced intercellular adhesion molecule-1 expression on pulmonary microvascular endothelial cells, MMP-9 activation, hypoxia-induced stimulation of pulmonary arterial cells, hyperosmolarity-induced IL-8 expression in bronchial epithelial cells, and enhanced eosinophil trafficking and survival.

Trifilieff et al. (2005 Brit J Pharmacol 144:1002-10) reported that CGH2466, a combined adenosine receptor antagonist, p38 MAPK and phosphodiesterase type 4 inhibitor showed potent *in vitro* and *in vivo* anti-inflammatory activities in diseases such as asthma and COPD. Underwood et al. (2000 Am J Physiol Lung Cell Mol Physiol 279:L895-L902) demonstrated that the potent and selective p38 MAPK inhibitor, SB239063, reduced proinflammatory cytokine production, including IL-1β, TNF-α, IL-6, and IL-8, which have been linked to airway fibrosis because of their ability to regulate fibroblast proliferation and matrix production; that leads to diminished neutrophil trafficking and activation in the lung. Earlier, the same compound was found capable of altering responses associated with chronic fibrosis induced by bleomycin. This inhibitory activity was selective for the α and β isoforms of the p38. The compounds and methods described herein are useful in the treatment of COPD.

### Pulmonary fibrosis

Pulmonary fibrosis also called idiopathic pulmonary fibrosis (IPF), interstitial diffuse pulmonary fibrosis, inflammatory pulmonary fibrosis, or fibrosing alveolitis, is an inflammatory lung disorder and a heterogeneous group of conditions characterized by abnormal formation of fibrous tissue between alveoli caused by alveolitis comprising an inflammatory cellular infiltration into the alveolar septae with resulting fibrosis. The effects of IPF are chronic, progressive, and often fatal. p38 MAPK activation has been demonstrated in the lung of patients with pulmonary fibrosis. A number of investigations about pulmonary fibrosis have indicated that sustained and augmented expression of some cytokines in the lung are relevant to recruitment of inflammatory cells and accumulation of extracellular matrix components followed by remodeling of the lung architecture. In particular, proinflammatory cytokines such as TNF-α and interleukin IL-1β were demonstrated to play major roles in the formation of pneumonitis and pulmonary fibrosis. In addition, profibrotic cytokines such as TGF-β and CTGF also play critical roles in the pathogenesis of pulmonary fibrosis. Matsuoka et al. (2002 Am J Physiol Lung Cell Mol Physiol 283:L103-L112) have demonstrated that a p38 inhibitor, FR-167653, ameliorates murine bleomycin-induced pulmonary fibrosis. Furthermore, pirfenidone (5-methyl-1-phenyl-2-(1H)-pyridone), a compound with combined anti-inflammatory, antioxidant and antifibrotic effects was found effective in experimental models of pulmonary fibrosis as well as in clinical studies (Raghu et al. 1999 Am J Respir Crit Care Med 159:1061-1069; Nagai et al. 2002 Intern Med 41:1118-1123; Gahl et al. 2002 Mol Genet Metab 76:234-242; Azuma et al. 2002 Am J Respir Crit Care Med 165:A729). The compounds and methods described herein are useful in the treatment of pulmonary fibrosis, such as IPF.

### Renal fibrosis

Irrespective of the nature of the initial insult, renal fibrosis is considered to be the common final pathway by which kidney disease progresses to endstage renal failure. Stambe et al. (2004 JAm Soc Nephrol 15:370-379) tested an inhibitor of the active (phosphorylated) form of p38, NPC 31169, developed by Scios Inc. (San Francisco, CA) in a rat model of renal fibrosis, and reported a significant reduction in renal fibrosis assessed by interstitial volume, collagen IV deposition, and connective tissue growth mRNA levels. The compounds and methods described herein are useful in the treatment of renal fibrosis.

### Leiomyoma

Uterine leiomyomas or fibroids are the most common pelvic tumors in women with no known long-term effective drug therapies available. Leiomyomas are characterized by increased cell proliferation and tissue fibrosis. Pirfenidone was tested on cell proliferation and collagen expression in cultured myometrial and leiomyoma smooth muscle cells, and was found to be an effective inhibitor of myometrial and leiomyoma cell proliferation (Lee et al. 1998 J Clin Endocrinol Metab 83:219-223). The compounds and methods described herein are useful in the treatment of leiomyomas.

### Endomyocardial fibrosis

Endomyocardial fibrosis (EMF) is a disorder characterized by the development of restrictive cardiomyopathy. EMF is sometimes considered part of a spectrum of a single disease process that includes Löffler endocarditis (nontropical eosinophilic endomyocardial fibrosis or fibroplastic parietal endocarditis with eosinophilia). In EMF, the underlying process produces patchy fibrosis of the endocardial surface of the heart, leading to reduced compliance and, ultimately, restrictive physiology as the endomyocardial surface becomes more generally involved. Endocardial fibrosis principally involves the inflow tracts of the right and left ventricles and may affect the atrioventricular valves, leading to tricuspid and mitral regurgitation. MAPK activation was shown to contribute to arrhythmogenic atrial structural remodeling in EMF. The compounds and methods described herein are useful in the treatment and/or prevention of endomyocardial fibrosis.

### Other inflammatory diseases

Many autoimmune diseases and diseases associated with chronic inflammation, as well as acute responses, have been linked to activation of p38 MAP kinase and overexpression or dysregulation of inflammatory cytokines. These diseases include, but are not limited to: rheumatoid arthritis; rheumatoid spondylitis; osteoarthritis; gout, other arthritic conditions; sepsis; septic shock; endotoxic shock; gram-negative sepsis; toxic shock syndrome; asthma; adult respiratory distress syndrome; chronic obstructive pulmonary disease; chronic pulmonary inflammation; inflammatory bowel disease; Crohn's disease; psoriasis; eczema; ulcerative colitis; pancreatic fibrosis; hepatic fibrosis; acute and chronic renal disease; irritable bowel syndrome; pyresis; restenosis; cerebral malaria; stroke and ischemic injury; neural trauma; Alzheimer's disease; Huntington's disease; Parkinson's disease; acute and chronic pain; allergic rhinitis; allergic conjunctivitis; chronic heart failure; acute coronary syndrome; cachexia; malaria; leprosy; leishmaniasis; Lyme disease; Reiter's syndrome; acute synoviitis; muscle degeneration, bursitis; tendonitis; tenosynovitis; herniated, ruptures, or prolapsed intervertebral disk syndrome; osteopetrosis; thrombosis; cancer; restenosis; silicosis; pulmonary sarcosis; bone resorption diseases, such as osteoporosis; graft-versus-host reaction; and auto-immune diseases, such as Multiple Sclerosis, lupus and fibromyalgia; AIDS and other viral diseases such as Herpes Zoster, Herpes Simplex I or II, influenza virus and cytomegalovirus; and diabetes mellitus.

Many studies have shown that reducing the activity of p38 MAP kinase, its upstream activators or its downstream effectors, either through genetic or chemical means, blunts the inflammatory response and prevents or minimizes tissue damage (see, e.g., English, et al. 2002 Trends Pharmacol Sci 23:40-45; and Dong et al. 2002 Annu Rev Immunol 20:55-72). Thus, inhibitors of p38 activity, which also inhibit excess or unregulated cytokine production and may inhibit more than a single pro-inflammatory cytokine, may be useful as anti-inflammatory agents and therapeutics. Furthermore, the large number of diseases associated with p38 MAP kinase-associated inflammatory responses indicates that there is a need for effective methods for treating these conditions.

Cardiovascular disease. Inflammation and leukocyte activation/infiltration play a major role in the initiation and progression of cardiovascular diseases including atherosclerosis and heart failure. Acute p38 mitogen-activated protein kinase (MAPK) pathway inhibition attenuates tissue damage and leukocyte accumulation in myocardial ischemia/reperfusion injury. The compounds and methods described herein are useful for treating cardiovascular disease.

Multiple sclerosis. Inflammation in the central nervous system occurs in diseases such as multiple sclerosis and leads to axon dysfunction and destruction. Both *in vitro* and *in vivo* observations have shown an important role for nitric oxide (NO) in mediating inflammatory axonopathy. p38 MAP kinase is activated by NO exposure and inhibition of p38 signalling was shown to lead to neuronal and axonal survival effects. OCM and IGF-1 reduced p38 activation in NO-exposed cortical neurons and improved axon survival in cultures exposed to NO, a process dependent on mitogen-activated protein kinase/extracellular signal-related kinase signalling. The compounds and methods described herein are useful for treating multiple sclerosis.

Primary graft nonfunction. Nonspecific inflammation is associated with primary graft nonfunction (PNF). Inflammatory islet damage is mediated at least partially by pro-inflammatory cytokines, such as interleukin-1β (IL-1β) and tumor necrosis factor-α (TNF-α) produced by resident islet macrophages. The p38 pathway is known to be involved in cytokine production in the cells of the monocyte-macrophage lineage. Inhibition of the p38 pathway by a chemical p38 inhibitor, SB203580, suppresses IL-1β and TNF-α production in human islets exposed to lipopolysaccharide (LPS) and/or inflammatory cytokines. Although IL-1β is predominantly produced by resident macrophages, ductal cells and islet vascular endothelial cells were found to be another cellular source of IL-1β in isolated human islets. SB203580 also inhibited the expression of inducible nitric oxide synthase (iNOS) and cyclooxygenase-2 (COX-2) in the treated islets. Furthermore, human islets treated with SB203580 for 1 h prior to transplantation showed significantly improved graft function. The compounds and methods described herein are useful for improving graft survival in clinical islet transplantation.

Acute renal injury. Cisplatin is an important chemotherapeutic agent but can cause acute renal injury. Part of this acute renal injury is mediated through tumor necrosis factor-α (TNF- α). Cisplatin activates p38 MAPK and induces apoptosis in cancer cells. p38 MAPK activation leads to increased production of TNF- α in ischemic injury and in macrophages. *In vitro,* cisplatin caused a dose dependent activation of p38 MAPK in proximal tubule cells. Inhibition of p38 MAPK activation led to inhibition of TNF- α production. *In vivo,* mice treated with a single dose of cisplatin developed severe renal dysfunction, which was accompanied by an increase in kidney p38 MAPK activity and an increase in infiltrating leukocytes. However, animals treated with the p38 MAPK inhibitor SKF86002 along with cisplatin showed less renal dysfunction, less severe histologic damage and fewer leukocytes compared with cisplatin+vehicle treated animals. The compounds and methods described herein are useful for preventing acute renal injury.

Periodontitis. The proinflammatory mediator bradykinin (BK) stimulates interleukin-8 (IL-8) production in human gingival fibroblasts *in vitro* and plays an important role in the pathogenesis of various inflammatory diseases including periodontitis. The specific p38 mitogen-activated protein kinase (MAPK) inhibitor SB 203580 reduced IL-8 production stimulated by the combination of BK and IL-1β as well as the IL-1 β-stimulated IL-8 production. The compounds and methods described herein are useful for treating or preventing periodontitis.

In some embodiments, the p38 inhibitors for any of the uses descrbed above are not selected from the group consisting of: 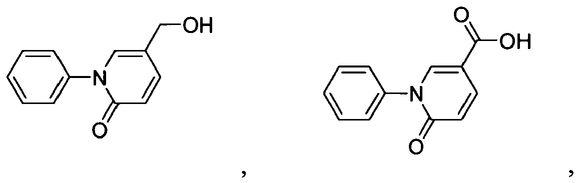 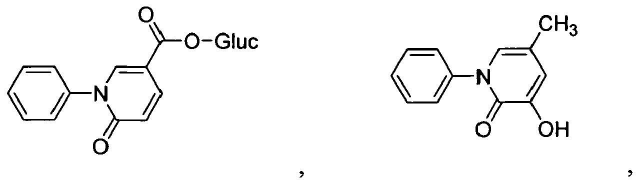 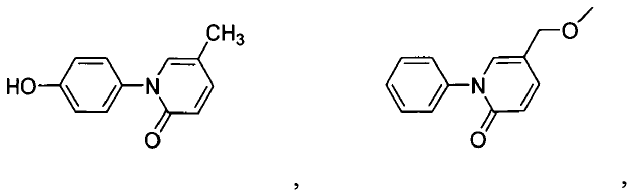 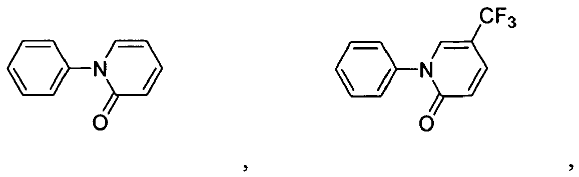 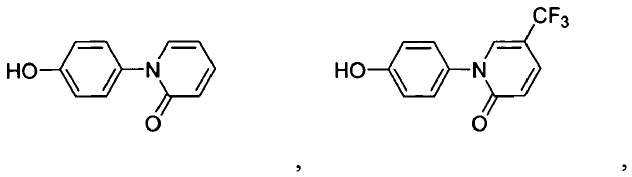 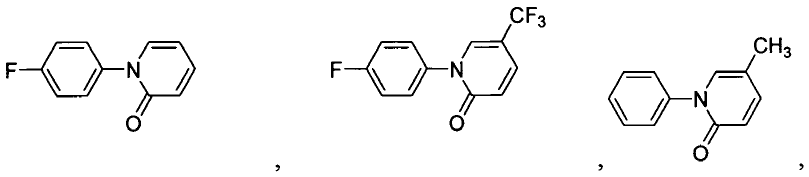 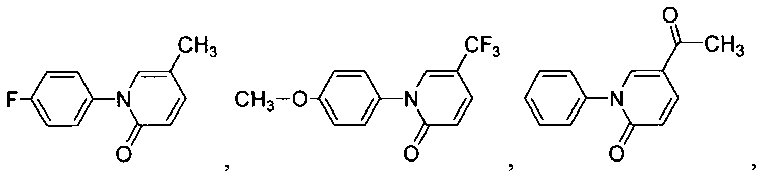 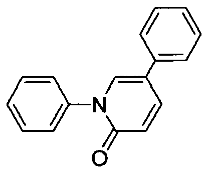 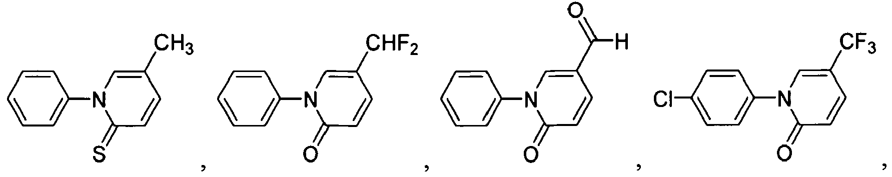 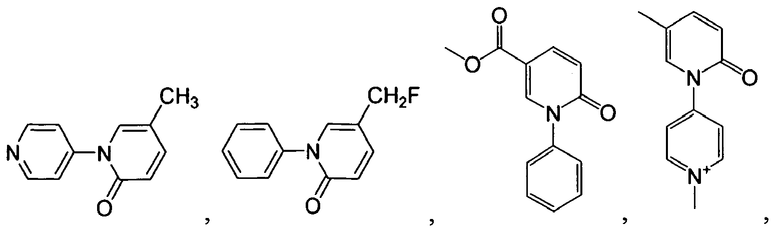 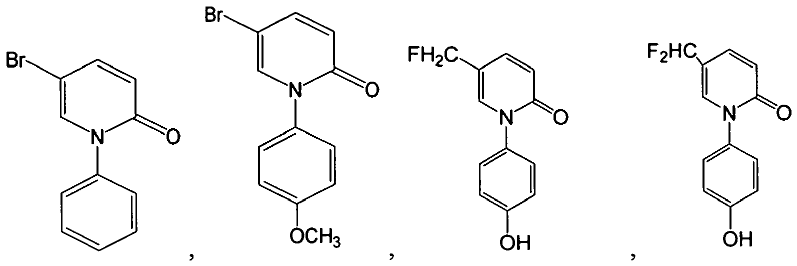 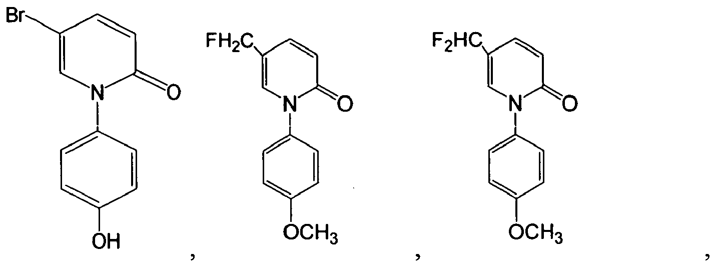 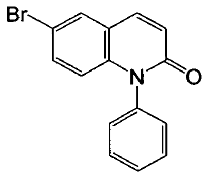 , and pirfenidone.

### Pharmaceutical Compositions

While it is possible for the compounds described herein to be administered alone, it may be preferable to formulate the compounds as pharmaceutical compositions. As such, in yet another aspect, pharmaceutical compositions useful in the methods of the invention are provided. More particularly, the pharmaceutical compositions described herein may be useful, *inter alia,* for treating or preventing inflammatory conditions, e.g., conditions associated with p38 activity or cytokine activity or any combination thereof. A pharmaceutical composition is any composition that may be administered *in vitro* or *in vivo* or both to a subject in order to treat or ameliorate a condition. In a preferred embodiment, a pharmaceutical composition may be administered *in vivo.* A subject may include one or more cells or tissues, or organisms. A preferred subject is a mammal. A mammal includes any mammal, such as by way of non-limiting example, cattle, pigs, sheep, goats, horses, camels, buffalo, cats, dogs, rats, mice, and humans. A highly preferred subject mammal is a human.

In an embodiment, the pharmaceutical compositions may be formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3 to a pH of about 11, preferably about pH 3 to about pH 7, depending on the formulation and route of administration. In alternative embodiments, it may be preferred that the pH is adjusted to a range from about pH 5.0 to about pH 8. More particularly, the pharmaceutical compositions may comprise a therapeutically or prophylactically effective amount of at least one compound as described herein, together with one or more pharmaceutically acceptable excipients. Optionally, the pharmaceutical compositions may comprise a combination of the compounds described herein, or may include a second active ingredient useful in the treatment or prevention of bacterial infection (e.g., anti-bacterial or anti-microbial agents).

Formulations, e.g., for parenteral or oral administration, are most typically solids, liquid solutions, emulsions or suspensions, while inhalable formulations for pulmonary administration are generally liquids or powders, with powder formulations being generally preferred. A preferred pharmaceutical composition may also be formulated as a lyophilized solid that is reconstituted with a physiologically compatible solvent prior to administration. Alternative pharmaceutical compositions may be formulated as syrups, creams, ointments, tablets, and the like.

The term "pharmaceutically acceptable excipient" refers to an excipient for administration of a pharmaceutical agent, such as the compounds described herein. The term refers to any pharmaceutical excipient that may be administered without undue toxicity.

Pharmaceutically acceptable excipients are determined in part by the particular composition being administered, as well as by the particular method used to administer the composition. Accordingly, there exists a wide variety of suitable formulations of pharmaceutical compositions (see, e.g., Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

The pharmaceutical compositions described herein may be formulated in any form suitable for the intended method of administration. When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as magnesium stearate, stearic acid or talc.

Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil.

In another embodiment, pharmaceutical compositions may be formulated as suspensions comprising a compound of the embodiments in admixture with at least one pharmaceutically acceptable excipient suitable for the manufacture of a suspension.

In yet another embodiment, pharmaceutical compositions may be formulated as dispersible powders and granules suitable for preparation of a suspension by the addition of suitable excipients.

Excipients suitable for use in connection with suspensions include suspending agents, such as sodium carboxymethylcellulose, methylcellulose, hydroxypropyl methylcellulose, sodium alginate, polyvinylpyrrolidone, gum tragacanth, gum acacia, dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycethanol), a condensation product of ethylene oxide with a partial ester derived from a fatty acid and a hexitol anhydride (e.g., polyoxyethylene sorbitan monooleate); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

The pharmaceutical compositions may also be in the form of oil-in water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, the pharmaceutical compositions may be in the form of a sterile injectable preparation, such as a sterile injectable aqueous emulsion or oleaginous suspension. This emulsion or suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,2-propane-diol.

The sterile injectable preparation may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids such as oleic acid may likewise be used in the preparation of injectables.

To obtain a stable water-soluble dose form of a pharmaceutical composition, a pharmaceutically acceptable salt of a compound described herein may be dissolved in an aqueous solution of an organic or inorganic acid, such as 0.3 M solution of succinic acid, or more preferably, citric acid. If a soluble salt form is not available, the compound may be dissolved in a suitable co-solvent or combination of co-solvents. Examples of suitable co-solvents include alcohol, propylene glycol, polyethylene glycol 300, polysorbate 80, glycerin and the like in concentrations ranging from about 0 to about 60% of the total volume. In one embodiment, the active compound is dissolved in DMSO and diluted with water.

The pharmaceutical composition may also be in the form of a solution of a salt form of the active ingredient in an appropriate aqueous vehicle, such as water or isotonic saline or dextrose solution. Also contemplated are compounds which have been modified by substitutions or additions of chemical or biochemical moieties which make them more suitable for delivery (e.g., increase solubility, bioactivity, palatability, decrease adverse reactions, etc.), for example by esterification, glycosylation, PEGylation, etc.

In a preferred embodiment, the compounds described herein may be formulated for oral administration in a lipid-based formulation suitable for low solubility compounds. Lipid-based formulations can generally enhance the oral bioavailability of such compounds.

As such, a preferred pharmaceutical composition comprises a therapeutically or prophylactically effective amount of a compound described herein, together with at least one pharmaceutically acceptable excipient selected from the group consisting of- medium chain fatty acids or propylene glycol esters thereof (e.g., propylene glycol esters of edible fatty acids such as caprylic and capric fatty acids) and pharmaceutically acceptable surfactants such as polyoxyl 40 hydrogenated castor oil.

In an alternative preferred embodiment, cyclodextrins may be added as aqueous solubility enhancers. Preferred cyclodextrins include hydroxypropyl, hydroxyethyl, glucosyl, maltosyl and maltotriosyl derivatives of α-, β-, and γ-cyclodextrin. A particularly preferred cyclodextrin solubility enhancer is hydroxypropyl-o-cyclodextrin (BPBC), which may be added to any of the above-described compositions to further improve the aqueous solubility characteristics of the compounds of the embodiments. In one embodiment, the composition comprises about 0.1 % to about 20% hydroxypropyl-o-cyclodextrin, more preferably about 1% to about 15% hydroxypropyl-o-cyclodextrin, and even more preferably from about 2.5% to about 10% hydroxypropyl-o-cyclodextrin. The amount of solubility enhancer employed will depend on the amount of the compound of the embodiments in the composition.

A pharmaceutical composition contains a total amount of the active ingredient(s) sufficient to achieve an intended therapeutic effect. More specifically, in some embodiments, the pharmaceutical composition contains a therapeutically effective amount (e.g., an amount of an SAPK-modulating compound that is effective in the prevention or treatment of the symptoms of an inflammatory disease or condition, wherein the compound exhibits an IC₅₀ in the range of about 100 µM to about 1000 µM, preferably about 200 µM to about 800 µM, for inhibition of the p38 MAPK). The total amounts of the compound that may be combined with the carrier materials to produce a unitary dosing form will vary depending upon the host treated and the particular mode of administration. Preferably, the compositions are formulated so that a dose of between 0.01 to 100 mg/kg body weight/day of an SAPK-modulating compound is administered to a patient receiving the compositions.

### EXAMPLE 1

Compounds are screened for the ability to inhibit ATF2 phosphorylation by p38 MAP kinase *in vitro.* The ability of compounds to inhibit ATF2 phosphorylation in this *in vitro* assay is correlated with the inhibition of p38 MAP kinase and TNFα expression *in vivo,* and is therefore an indicator of potential *in vivo* therapeutic activity (Raingeaud, J. et al. 1995 J. Biol. Chem. 270:7420-7426; Brinkman, M.N., et al. 1999 J Biol. Chem. 274:30882-30886; and Fuchs, S.Y. et al. J Biol. Chem. 275:12560-12564,2000).

All kinases and the substrate ATF2 are acquired from Upstate (Charlottesville, VA). p38 MAP Kinases are recombinant human full-length proteins with an amino-terminal GST fusion, expressed in and purified from *E*. *coli.* ATF2 is a GST fusion protein containing amino acids 19-96 of human ATF2 expressed in *E*. *coli.* All proteins are aliquoted and stored at -80°C.

p38 MAP kinase assays are performed using an assay buffer containing 25 mM HEPES, pH 7.5, 10 mM MgCl₂, 2 mM DTT, 20 mM β-glycerophosphate, 0.1 mM Na₃VO₄, 40 µM ATP and 1.25 µM of ATF2, together with 6 ng of p38α protein, 12 ng p38β protein, 1.5 ng p38γ, or 0.4 ng JNK2α2. Compounds are serially diluted in DMSO and 2 µL of test compound at various concentrations is used. The vehicle control receives DMSO only.

Test compounds are pre-incubated with 20 µl of enzyme in kinase buffer (25 mM HEPES, pH 7.5, 10 mM MgCl₂, 2 mM DTT, 20 mM β-glycerophosphate and 0.1 mM Na₃VO₄) at room temperature for 15 minutes. Reactions are initiated by addition of 30 µl substrate solution to yield a final concentration of 40 µM ATP and 1.25 µM ATF2 in kinase buffer. The reactions are incubated for 30 minutes at 37°C and terminated by the addition of 18 µl of 200 mM EDTA. An ELISA method is used to measure the phosphorylation of ATF2 at Thr 69. High binding 96-well plates are coated with 50 µl of kinase reaction for 1 hour at 37°C. The coated plates are washed with 200 µl washing buffer (25 mM Tris HCl, pH 8.3, 192 mM glycine, 0.1% SDS and 0.05% Tween-20) three times. The plates are then washed three times with SuperBlock in TBS (Pierce, 37535). After blocking, plates are incubated with 50 µl of rabbit anti-phospho-ATF2 antibody (Cell Signaling, 9221L, 1:500) for 30 minutes at 37°C.

Plates are washed three times with washing buffer prior to incubation with 50 µl HRP-conjugated goat anti-rabbit antibody (Cell Signaling, 7074, 1:500) for 30 minutes at 37°C. Plates are then washed three times with washing buffer before incubation with 50 µl of Ultra TMB-ELISA (Pierce, 34028) for 8 minutes at room temperature. Finally, 50 µl of phosphoric acid (1 M) is added to stop reactions and plate absorbance is read at 450 nm on a SpectraMax 250 plate reader.

The compounds inhibit the phosphorylation of ATF2 in this *in vitro* assay. Preferred compounds exhibit IC₅₀ values of less than about 100 µM, preferably less than about 1 µM. ,

### EXAMPLE 2

Compounds are screened for the ability to inhibit TNFα release from THP-1 cells stimulated with lipopolysaccharide (LPS) *in vitro.* The ability of compounds to inhibit TNFα release in this *in vitro* assay is correlated with the inhibition of p38 activity and TNFα, and is therefore an indicator of potential *in vivo* therapeutic activity (Lee J. C. et al. 1993 Ann. N.Y. Acad. Sci. 696:149-170; and1994 Nature 372:739-746).

THP- I cells from ATCC (TIB202) are maintained at 37°C, 5% CO₂ in RPMI 1640 media (MediaTech, Herndon, VA) containing 4.5 g/L glucose, supplemented with 10% fetal bovine serum, 1% penicillin/streptomycin and 50 µM β-mercaptoethanol.

Test compounds are initially dissolved in RPMI media with 1% DMSO (v/v). Compounds are then serially diluted in RPMI media for all subsequent dilutions. The assay is performed under sterile conditions. THP-1 cells at a culture density of 6-8 x 10⁵ cells/ml are collected and resuspended in the RPMI media at 10⁶ cells/ml. 100 µl of resuspended cells are added to each well, which contain 100 µl of a test compound. Test compounds are prepared at twice the final concentration. Final DMSO concentration is no more than 0.5 % (v/v). Cells are preincubated with compound for 60 minutes at 37°C, 5% CO₂ prior to stimulation with lipopolysaccharide (LPS) (Sigma L-2880, 4 mg/ml stock in PBS). The final LPS concentration in each well is 10 or 30 µg/ml for TNFα and IL-1β release, respectively. Unstimulated control cell suspensions receive PBS vehicle only. Cell mixtures are incubated for 18 or 48 hours for TNFα and IL-1β release, respectively. 150 µl of supernatants are taken and transferred to a fresh plate and stored at -20°C until farther analysis. TNFα and IL-1β levels are measured using ELISA kits. A Luminescence is used as the plate reader. Analysis is performed by non-linear regression to generate a dose response curve. The calculated IC₅₀ value is the concentration of the test compound that causes a 50 % decrease in TNFα or IL-1β levels.

Compounds inhibit the release of TNFα, IL-1β or both TNFα, and IL-1β in this *in vitro* assay. Preferred compounds exhibit IC₅₀ values for TNFα and/or IL-1β of less than about 100 µM, preferably less than about 1 µM.

### EXAMPLE 3

Compounds are screened for the ability to inhibit TNFα release from primary human peripheral blood mononuclear cells (PBMC) stimulated with lipopolysaccharide (LPS) *in vitro.* The ability of compounds to inhibit TNFα release in this *in vitro* assay is correlated with the inhibition of p38 activity and is therefore an indicator of potential *in vivo* therapeutic activity (2002 Osteoarthritis & Cartilage 10:961-967; and Laufer, S.A. and Wagner, G.K. 2002 J Med. Chem. 45: 2733-2740).

Human peripheral blood mononuclear cells (PBMC) are isolated by differential centrifugation through a Ficoll-HyPaque density gradient from pooled serum of 3-8 individual blood donors. Isolated PBMC contain approximately 10% CD-14 positive monocytes, 90% lymphocytes and <1% granulocytes and platelets. PBMC (10⁶ /ml) are cultured in polystyrene plates and stimulated with lipopolysaccharide (LPS; 50 ng/ml; Sigma, St. Louis, MO) in the presence and absence of the test compound in serial dilutions, in duplicate, for 24 hr at 37°C in GIBCO™ RPM1 medium (Invitrogen, Carlsbad, CA) without serum. The TNFα level in cell supernatants is determined by ELISA using a commercially available kit (MDS Panlabs #309700).

Preferred compounds inhibit the release of TNFα in this assay with an IC₅₀ value of less than about 100 µM, preferably less than about 1 µM.

### EXAMPLE 4

Compounds are screened for the ability to inhibit the release of TNFα in an *in vivo* animal model (See, e.g., Griswold D. E. et al. 1993 Drugs Exp. Clin. Res. 19:243-248; Badger, A.M. et al. 1996 J. Pharmacol. Exp. Ther. 279:1453-1461; Dong, C. et al. 2002 Annu. Rev. Immunol. 20:55-72 (and references cited therein); Ono, K. and Han, J. 2000 Cellular Signalling 12:1-13 (and references cited therein); and Griffiths, J.B. et al. 1999 Curr. Rheumatol. Rep. 1:139-148).

Without being bound by any particular theory, it is believed that inhibition of TNFα in this model is due to inhibition of p38 MAP kinase by the compound.

Male Sprague-Dawley rats (0.2 - 0.35 kg) are randomly divided into groups of six or more and are dosed intravenously by infusion or bolus injection, or are dosed orally with test compounds in a suitable formulation in each case. Thirty minutes following end of infusion or bolus injection, and 1-2 hr following oral administration, lipopolysaccharide *E*. *coli*/0127:B8 (0.8 mg/kg) is administered IV. Blood samples are collected 1.5 hours post-treatment with LPS. Serum TNFα levels are determined using the ELISA kit from Biosource (KRC3011C) and compared to that from vehicle-treated control.

Preferred compounds inhibit the release of TNFα in this *in vivo* assay. Preferred compounds exhibit an ED₅₀ value of less than 500 mg/kg, preferably less than 400 mg/kg, preferably less than 200 mg/kg, preferably less than 100 mg/kg, more preferably, less than 50 mg/kg, more preferably, less than 40 mg/kg, more preferably, less than 30 mg/kg, more preferably, less than 20 mg/kg, more preferably, less than 10 mg/kg.

The methods of determining the IC₅₀ of the inhibition of p38 by a compound include any methods known in the art that allow the quantitative detection of any of the downstream substrates of p38 MAPK as described above. Therefore, these methods additionally include but limited to detection of expression of genes known to be regulated by p38 either individually, or by gene arrays.

### EXAMPLE 5

### Kinase Assay

The activity of the P38 kinase isoforms P38γ and P38α is determined by phosphorylation of ATF-2 in presence of ³²P-γ-ATP. The incorporation of ³²P into ATF-2 in the presence or absence of inhibitors is determined. The compounds described herein are tested for inhibition of P38γ and P38α kinase activity in this biochemical assay. The compounds are solubilized in water or DMSO and tested at different concentrations from 0 to 10 mM using the appropriate solvent for dilutions and as vehicle control. The enzymes P38γ and P38α are obtained as activated and purified recombinant protein (Upstate, Charlottesville,VA). The activated enzyme is used at 24.8 nM in the final reaction. The enzymes are diluted prior to the reaction in the following buffer (1M HEPES, pH 7.4, 500mM DTT, 1% Triton X-100 and 10mg/ml BSA). The reaction is performed in the following solution that is prepared as a two fold stock solution (1M HEPES, pH 7.4, 500mM DTT and 1% Triton X-100) and non-radioactive ATP is present in the reaction at 6.25 µM ATP (Cell Signaling, Beverly, MA). To determine the phosphorylation of ATF-2, ³²P-γ-ATP 3000Ci/mmol is added to each reaction at a concentration of 7.5 µM. ATF-2 (Cell Signaling, Beverly, MA) as a kinase substrate is used at 3 µM. As a first step in assembling the enzyme reaction, activated kinase and inhibitor or the appropriate vehicle control are added to reaction buffer and incubated for 30 min at room temperature. The kinase reaction is initiated by the addition of ATF-2 and ATP mixture. The final volume for each reaction is 20 µl and performed at room temperature for 30 minutes. After the 30 minutes of incubation 80ul of Laemmlie buffer is added. Subsequently 20% of the reaction is separated on a SDS -Page (BioRad, Hercules, CA) under reducing conditions. After the electrophoresis, the gel is exposed to a ³²P-intensifying screen over night at room temperature and analyzed using a phosphoimager, Storm System (Amersham Biosciences, Piscataway, NJ). The signal obtained is quantified after background correction and calculated as percent inhibition using the uninhibited kinase activity with the vehicle control as 0% inhibition. The kinase activity, in the presence of different inhibitor concentrations, is plotted using Kaleidagraph (Synergy Software, Reading, PA) to determine the EC₅₀ for each compound and tested P38 kinase.

### Inhibition of TNFα induction

THP-1 (ATCC, Rockville, MD) is grown under regular tissue culture conditions as recommended by ATCC. 18 hours prior to the experiment, cells were plated in a 96 well format in regular culture media containing 1% serum and 0.25 ml culture volume at a density of 500,000 cells per well. The compound is added to each well in triplicates and the appropriate solvent control is included in each assay. The P38 inhibitor SB203850 at 1 µM/ml (Upstate, Waltham, MA) is included as a positive control in each assay. For the induction of TNFα expression, 1 µg/ml LPS is added to each well 30 minutes post compound addition. Following a 4 hour incubation under tissue culture conditions the cells are sedimented by centrifugation (10 min, 1000rpm, Beckman table top centrifuge) and a fraction of the cell free supernatant is collected and used in a tenfold dilution for the quantification in the TNFα specific ELISA (R&D Systems, Minneapolis, MN). The TNFα ELISA is performed according to the directions provided by the manufacturer. The TNFα is detected in pg/ml and plotted as fractional activity normalized to the TNFα expression in the solvent control.

### Compound toxicity testing in a cell based assay

The release of LDH as result of a disrupted cell membrane is applied as a measure of cell toxicity. LDH is detected by its enzymatic activity using a commercially available diagnostic kit (Roche Diagnostics, Cat# 1 644 793). THP-1 cells are used for determination of cell toxicity for consistency with the induced TNFα expression in the previous experiment. As previously described for testing of inhibition of TNFα induction, cells are cultured in a 96 well format under 1% serum and regular tissue culture conditions. The compounds are added at different concentrations in triplicate. The appropriate solvent control is used in each assay. After compound addition the cells are cultured 18 hours under regular tissue culture conditions. After this incubation period, the positive control is initiated by adding Triton-x-100 (2% v/v) to untreated cells and incubated for an additional 10 minutes for complete cell lysis. Subsequently the cells are sedimented by centrifugation and a fraction of the supernatant removed and analyzed for LDH enzyme activity according to the manufacturer's instructions. The data are displayed as % cell toxicity normalized to the Triton-X-100 lysed cells as 100% cell toxicity.

### EXAMPLE 6

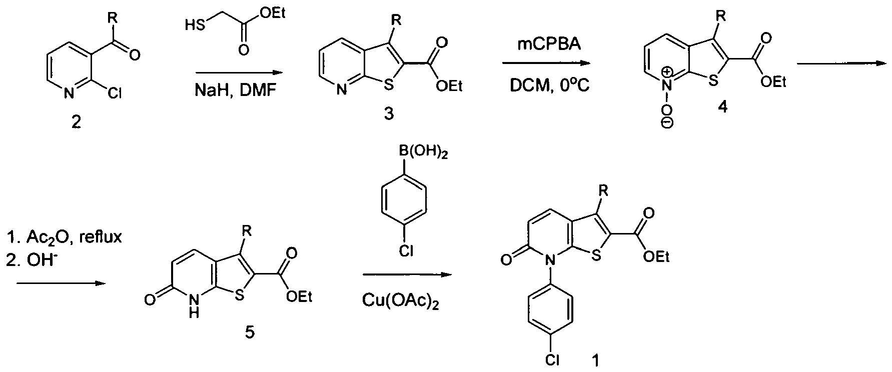

The compound of formula (1) is synthesized as depicted immediately above. R-substituted 3-acylpyridine (compound 2), which is obtained from commercial sources or prepared via known procedures, is reacted with ethyl mercapto acetate anion to form the thieno[2,3-b]pyridine derivative (compound 3). Compound 3 is oxidized to the N-oxide (compound 4), which is converted to the pyridinone (compound 5) upon treatment with acetic anhydride followed by subsequent hydrolysis. Compound 5 is N-arylated using known procedures, such as shown in scheme 1 using 4-chlorophenyl boronic acid in the presence of copper acetate. Compound 1 is an example of a compound of Genus I. Other compounds of Genus I may be synthesized in a similar fashion.

### EXAMPLE 7

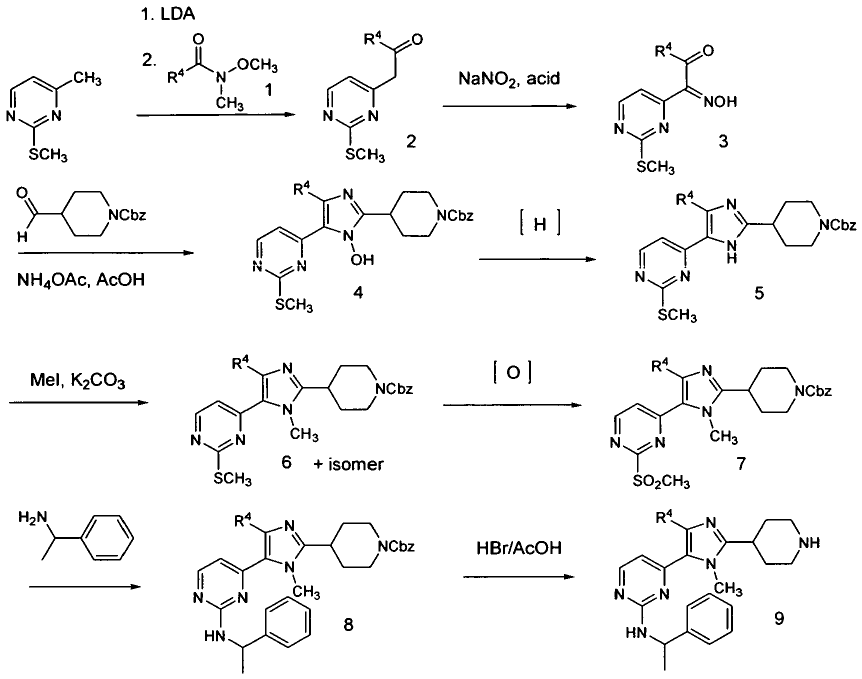

The compound of formula (9) is synthesized as depicted immediately above. Deprotonation of 2-thiomethyl-4-methylpirimidine with lithium diisopropylamide followed by a treatment with suitable N,O-dimethylhydroxamide (compound 1) provides the corresponding ketone (compound 2). The latter is converted to the alpha-oximoketone (compound 3) by reaction with sodium nitrite in acetic acid. Condensation of compound 3 with N-benzyloxycarbonyl (Cbz)-protected piperidine-4-carbaldehyde gives the N-hydroxyimidazole (compound 4), which is transformed to the N-methyl-imidazole (compound 6) by reduction to form the imidazole intermediate (compound 5) under usual conditions followed by methylation. After oxidation to form the sulfone (compound 7), the methylsulfonyl group is displaced with amine to furnish the aminopyrimidine derivate (compound 8). The final deprotection of piperidine nitrogen under standard conditions, such as HBr in acetic acid, provides the target substituted imidazole (compound 9). Compound 9 is an example of a compound of Genus II. Other compounds of Genus II may be synthesized in a similar fashion.

### EXAMPLE 8

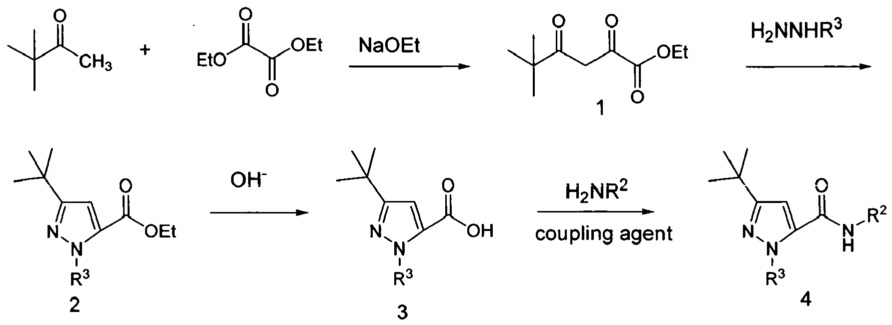

The compound of formula (4) is synthesized as depicted immediately above, in accordance with known methods (see J. Org. Chem., 62, 17, 5908-5919). Pinacolone anion is reacted with ethyl oxalate to give the ethyl ester of 5,5-dimethyl-2,4-dioxohexanoic acid (compound 1). Reaction of compound 1 with the appropriately substituted hydrazine affords the pyrazole (compound 2), which is converted to the target compound 4 by the usual procedures. Compound 4 is an example of a compound of Genus III. Other compounds of Genus III may be synthesized in a similar fashion.

### EXAMPLE 9

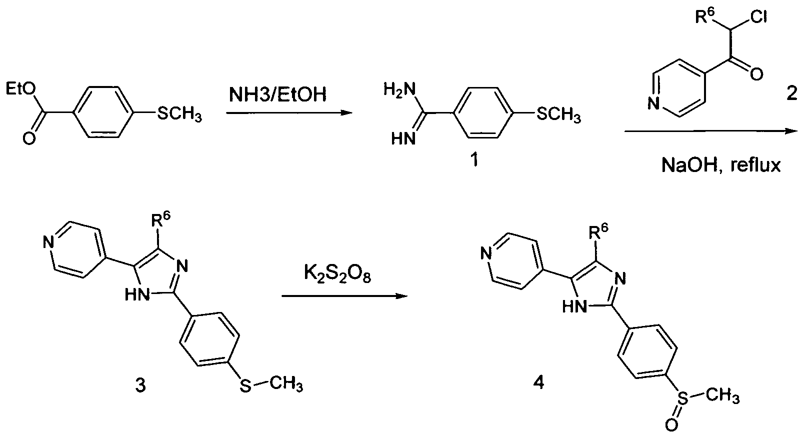

The compound of formula (4) is synthesized as depicted immediately above. A benzamidine (compound 1), prepared by treatment of ethyl 4-methylthoimethylbenzoate with ethanolic ammonia, is reacted with alpha-chloroketone (compound 2) to furnish the imidazole (compound 3). The target compound 4 is obtained after oxidation of the intermediate 3 under standard conditions, for instance with potassium persulphate as depicted. Compound 4 is an example of a compound of Genus IV. Other compounds of Genus IV may be synthesized in a similar fashion.

### EXAMPLE 10

*Introduction of short hairpin RNAs (shRNAs) targeting p38*α *or p38γ into cultured cells.* MISSION ™ TRC shRNA lentiviral particles were obtained from Sigma Aldrich (St. Louis, MO). These self-inactivating replication incompetent viral particles are designed to express shRNAs from a U6 promoter and allow for puromycin selection of infected cells. The shRNAs used in this study are designed to target human p38γ (lentiviral particles containing coding regions with SEQ IDs Nos: 1-4), human p38α (lentiviral particles containing coding regions with SEQ IDs No.: 5-8). A first control lentiviral particle encodes a shRNA that carries at least 5 mismatches to all known human and mouse genes and does not target any known human or mouse gene. A second control lentiviral particle does not express an shRNA. A third control lentiviral particle designed to express green fluorescent protein (GFP) was used to optimize infection conditions.

HFL1 cells (ATCC, Rockville, MD) were maintained in at 37°C and 5% CO2 in F12K media (MediaTech, Herndon, VA) supplemented with L-Glutamate, 10% fetal calf serum, sodium bicarbonate (1.5g/L), and penicillin/streptomycin. Cells were plated in 24 well plates 1 day prior to infection and were approximately 2/3 confluent at the time of infection. For infection, media was removed and replaced with fresh media containing 0-12 µg/mL hexadimethrine bromide (Sigma Aldrich, St. Louis, MO) followed by addition of lentiviral particles at a multiplicity of infection (MOI) of 1-5. Twenty-four hours later, infection media was removed and replaced with fresh media. For all steps post infection, cells were passaged as necessary to maintain stock between 5 and full confluence. Between 1 and 3 days post-infection cells were placed under puromycin selection with a final concentration of 0.25-1 µg/mL, which was defined based on the puromycin sensitivity of the uninfected cells. Selection was maintained for 5-10 days and the progress of selection was assessed by observing cells transfected with a lentivirus designed to express GFP as well as the loss of viability in uninfected cells. HFL1 cells were mock infected (no virus) or infected with a lentivirus designed to confer puromycin resistance and express one of the following: an shRNA targeting human p38α (SEQ ID NOS:5-8), an shRNA targeting human p38γ (SEQ ID NOS:1-4), an shRNA that does not target a known human or mouse gene, nothing (control virus), or green fluorescent protein.

NIH3T3 cells (ATCC. Rockville, MD) were maintained in DMEM media (MediaTech, Herndon, VA) supplemented with L-Glutamate, 10% fetal calf serum, sodium bicarbonate (1.5g/L), and penicillin/streptomycin. Cells were infected and subjected to puromycin selection as described above. NIH3T3 cells were mock infected (no virus) or infected with a lentivirus designed to confer puromycin resistance and express one of the following: an shRNA targeting mouse p38α (SEQ ID NOS:5-8), an shRNA targeting mouse p38γ (SEQ ID NOS:1-4), an shRNA that does not target a know human or mouse gene, nothing (control virus), or green fluorescent protein.

### EXAMPLE 11

*Isolation of mRNA and determination of mRNA levels using quantitative PCR.* RNA from ∼15,000 to 150,000 cells was obtained using the RNaqueous 96 RNA isolation kit (Ambion, Austin, TX) according to the manufacturer's instructions. RNA was converted to cDNA using TaqMan® Reverse Transcription Reagents (Applied Biosystems, Branchburg, NJ) using random hexamer primers according to the manufacturer's instructions. Quantitative PCR reactions were performed in triplicate on a Applied Biosystems 7900 HT Sequence Detection System (Applied Biosystems, Branchburg, NJ) using the cDNA obtained above, a commercially available probe primer set (TaqMan® Gene Expression Arrays, Applied Biosystems) and TaqMan® Universal PCR Master Mix (Applied Biosystems, Branchburg, NJ) according to the manufacturer's instructions. Three plates were run for HFL1 cells using probe/primer sets designed to amplify human glyceraldehyde-3-phosphate dehydrogenase (GAPDH; endogenous control), human p38α, and human p38γ. Three plates were run for NIH3T3 cells using probe/primer sets deigned to amplify mouse glyceraldehyde-3-phosphate dehydrogenase (GAPDH; endogenous control), mouse p38α, and mouse p38γ. Data were analyzed using a ΔΔCt method where the level of input RNA was normalized using glyceraldehydes-3-phosphate dehydrogenase as an endogenous control. Threshold cycle (Ct) values were obtained from the geometric phase of an amplification plot. Relative levels of gene expression were determined using the following equation: $ΔΔCt = \left(Average {Ct}_{analyte GAPDH}-Average {Ct}_{control GAPDH}\right) - \left(Average {Ct}_{analyte p ⁢ 38}-Average {Ct}_{control p ⁢ 38}\right)$
Where
Average Ct_{analyte GAPDH} is the average Ct value for an analyte (lentiviral treated) cell line with a GAPDH probe primer set
Average Ct_{control GAPDH} is the average Ct value for control cells (untreated) with a GAPDH probe primer set
Average Ct_{analyte p38} is the average Ct value for an analyte (lentiviral treated) cell line with a p38 (α or γ) probe primer set
Average Ct_{control p38} is the average Ct value for control cells (untreated) with a p38 probe primer set

Data are reported as the relative p38 mRNA level in lentivirus treated cells compared to untreated cells when the input RNA levels are corrected using the GAPDH endogenous control. The observed p38 mRNA levels for selected shRNA constructs are provided in Table 1 at Example 12 below.

### EXAMPLE 12

*Assay of collagen levels.* Normal HFL1 cells or cells infected with lentivirus and selected with puromycin were grown to confluence at 37°C and 5% CO₂ in F12K media (MediaTech, Herndon, VA) supplemented with L-Glutamate, 10% fetal calf serum, sodium bicarbonate (1.5g/L), and penicillin/streptomycin. For collagen assays, cells were plated in 6-well plates at a density of ∼6 x 10⁵ cells per well in 2 mL/well of starvation media (F12K media supplemented with L-Glutamate, 0.1 % fetal calf serum, sodium bicarbonate (1.5g/L), and penicillin/streptomycin). 16-20 hours later, the media is removed and replaced with fresh starvation media supplemented with 20 µg/mL ascorbic acid (EM Science, Gibbstown, NJ) and 10uM L-proline (Calbiochem, L Jolla, CA). One hour following this media change cells were treated with 5ng/mL (10 ng/well) transforming growth factor-β1 (TGF-β; Chemicon/Millipore, Billerica, MA or Sigma Aldrich, St. Louis, MO). 40-48 hours after addition of TGF-β cell media was removed and cells were lysed into 250 µL phosphate buffered saline (PBS) supplemented with 1.25% Triton X-100 (Fisher, Fair Lawn, NJ) and complete proteinase inhibitor cocktail (Roche, Mannheim, Germany) Cells were scraped from the plate using a cell scraper or pipette tip and the lysate frozen at -20°C.

NIH3T3 cells and NIH3T3 cells infected with lentivirus and selected with puromycin were treated in a similar manner except that F12K media was replaced by DMEM (Mediatech, Herndon, VA). The DMEM media was supplemented with additional components as with F 12K above.

Harvested cells were thawed to ice. 200 µL of the sample was mixed with an equal volume of 0.5M acetic acid in a 1.7 mL microfuge tube. Collagen concentrations were determined using the Sircol Soluble Collagen Assay (Biocolor Ltd,Newtonabbey, Northern Ireland) using a variation of the manufacturer's protocol. Briefly, one milliliter of Sircol dye reagent was added and the sample was shaken for 30-60 minutes (the length of time was constant for all samples in an experiment). The sample was then centrifuged for 30 min at ∼10,000 x g in a model tabletop microcentrifuge. The unbound dye was removed from the pellet with a pipette tip using care to avoid disturbing the pellet. The sample was centrifuged for an additional 4 minutes and residual unbound dye was carefully removed from the pellet with a pipette tip. 200 µL of the manufacturer's alkali reagent was then added and the samples were shaken until the precipitate dissolved. A standard curve was created by using known amounts of collagen standard (Biocolor Ltd,Newtonabbey, Northern Ireland) in the protocol above. 100 µL of standard or sample was added to a clear 96 well plate and the sample aborbance at 540 nm was determined using a µQuant spectrophotometer (Bio-Tek, Winooski, VT).

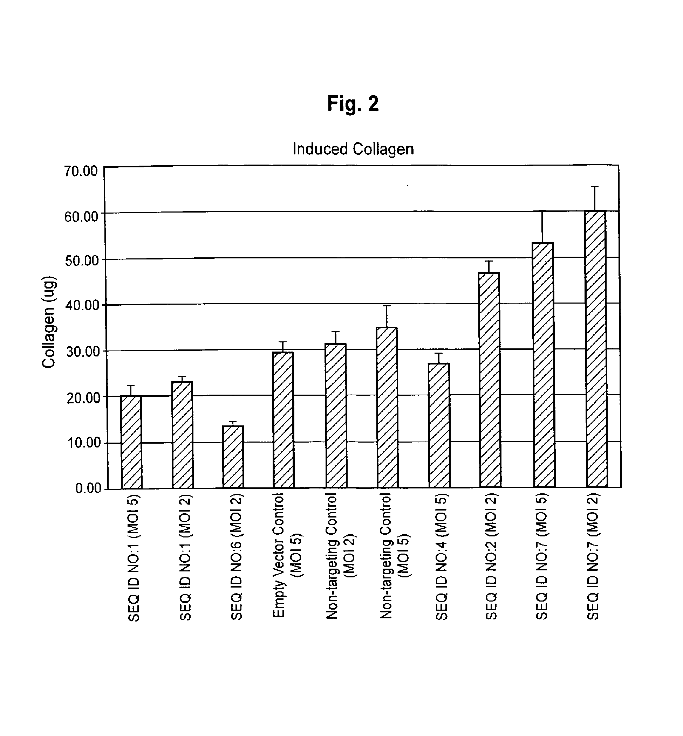

As can be seen in Table 1 below and Figures 1 and 2, decreased expression of p38α or p38γ resulted in decreased levels of collagen, and increased levels of p38α and p38γ resulted in increased levels of collagen. Thus, these data confirm that inhibiting, for example, p38γ, can result in decreased levels of collagen, and, therefore, decreased fibrosis.

**Table 1. Expression of p38 mRNA and collagen with different shRNA constructs.**

| **Sequence** | **multiplicity of infection (MOI)** | **collagen (µg) uninduced** | **standard deviation** | **collagen (µg) TGF-β induced** | **standard deviation** | **relative p38γ** | **relative p38α** |
|---|---|---|---|---|---|---|---|
| SEQ ID NO:1 | 5 | 9.91 | 2.61 | 19.76 | 2.42 | 0.5 | 1.15 |
| SEQ ID NO:1 | 2 | 10.60 | 2.36 | 22.91 | 1.18 | 0.52 | 1.36 |
| SEQ ID NO:6 | 2 | 12.87 | 0.72 | 13.18 | 1.09 | 1.37 | 0.59 |
| Empty Vector Control | 5 | 19.75 | 3.15 | 29.33 | 2.45 | 1.35 | 1.39 |
| Non-targeting control | 2 | 21.11 | 1.68 | 31.18 | 2.78 | 1.04 | 1.2 |
| Non-targeting control | 5 | 20.56 | 1.85 | 34.67 | 4.95 | 1.19 | 1.19 |
| SEQ ID NO:4 | 5 | 22.59 | 1.35 | 26.73 | 2.31 | 1.15 | 1.28 |
| SEQ ID NO:2 | 2 | 25.61 | 2.54 | 46.74 | 2.73 | 1.38 | 1.58 |
| SEQ ID NO:7 | 5 | 26.47 | 1.57 | 53.20 | 7.00 | 0.96 | 2.27 |
| SEQ ID NO:7 | 2 | 34.49 | 3.92 | 60.04 | 5.45 | 1.38 | 1.14 |

### EXAMPLE 13

Several compounds were evaluated for inhibition at p38α and p38γ MAPK isoforms. IC₅₀ values were determined using the assays described in Example 5 of WO 2006/122154, which is incorporated herein by reference in its entirety. The results are listed in Table 2.

**Table 2. p38 inhibitory effect of selected compounds.**

| 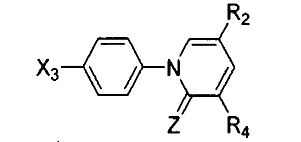 | | | | p38α IC₅₀ (µM) | p38γ IC₅₀ (µM) |
|---|---|---|---|---|---|
| **X₃** | **R₂** | **R₄** | **Z** | | |
| -H | -H | -H | O | 4000 | 140 |
| -OH | -CH₃ | -H | O | 500 | 127 |
| -H | -glucuronide | -H | O | 5000 | 1600 |
| -H | -CH₂OCH3 | -H | O | NC | NC |
| -H | -CH₃ | -OH | O | 200 | 800 |
| -F | -CH₃ | -H | O | NC | 410 |
| -OCH₃ | -CF₃ | -H | O | 6000 | 220 |
| -COCH₃ | -H | -H | O | 3300 | 1200 |
| -H | -CH₃ | -H | S | 200 | 700 |
| See note 1 | CH₃ | -H | O | NC | NC |
| -H | -Br | -H | O | 2600 | 90 |
| -OCH₃ | -Br | -H | O | 2100 | 31 |
| -OCH₃ | -CH₂F | -H | O | 1200 | 15 |
| -OCH₃ | -CHF₂ | -H | O | 5000 | 270 |
| -H | See note 2 | -H | O | 3800 | 46 |
| -H | CO₂CH₃ | -H | O | 8700 | 900 |
| Pirfenidone | | | | 1800 | 630 |

| | | | | | |
|---|---|---|---|---|---|
| ¹ The aryl group attached to the 2-pyridone nitrogen is an N-methylpyridinium moiety ² A bromoaryl group is fused to the 5 and 6 positions of a 2-pyridone ring NC (Not Calculated) refers to a value beyond the upper limit of quantitation, and thus too large to reliably assign | | | | | |

While the present invention has been described in some detail for purposes of clarity and understanding, one skilled in the art will appreciate that various changes in form and detail can be made without departing from the true scope of the invention. All figures, tables, appendices, patents, patent applications and publications, referred to above, are hereby incorporated by reference.

### Aspects of the Invention:

1. A compound of the formula: 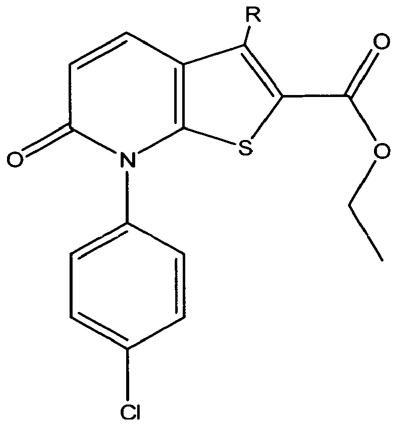 wherein R is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C₆ or ₁₀ aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.
2. A compound of the formula: 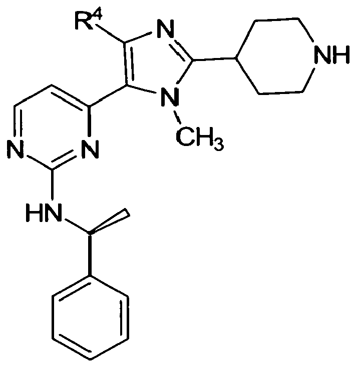 wherein R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.
3. A compound of the formula: 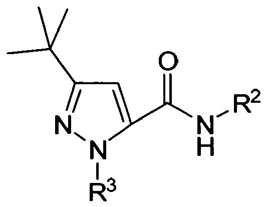 wherein R² and R³ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;
   wherein the compound is not 1-(5-tert-Butyl-2-p-tolyl-2H-pyrazol-3-yl)-3-[4-(2-morpholin-4-yl-ethoxy)naph- thalen-1-yl]urea (BIRB 796).
4. A compound of the formula: 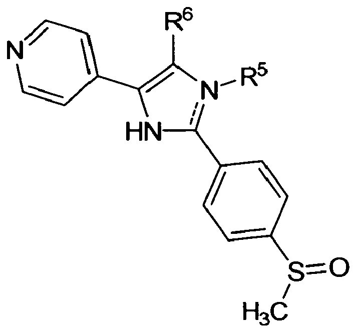 wherein R⁵ and R⁶ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C₆ or ₁₀ aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;
   wherein the compound is not 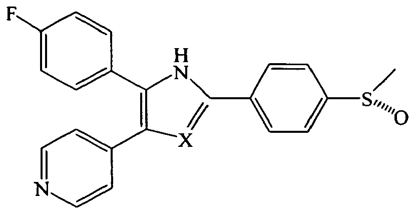 where X is N.
5. A compound of the formula: 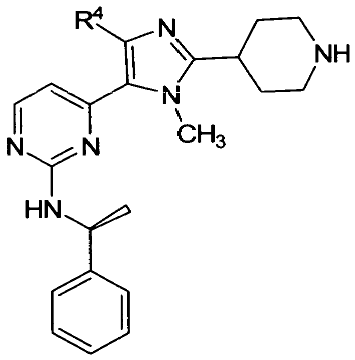 wherein, upon binding of the compound to p38γ, R⁴ is a group positioned not less than 5 Å and not more than 25 Å distant from a p38γ Met109 sulfhydryl moiety.
6. The compound of aspect 5 wherein R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C₆ or ₁₀ aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.
7. A method of treating or preventing an inflammatory or fibrotic disease in an individual, the method comprising administering to the individual an effective amount of the compound of aspect 1.
8. A method of treating or preventing an inflammatory or fibrotic disease in an individual, the method comprising administering to the individual an effective amount of the compound of aspect 2.
9. A method of treating or preventing an inflammatory or fibrotic disease in an individual, the method comprising administering to the individual an effective amount of the compound of aspect 3.
10. A method of treating or preventing an inflammatory or fibrotic disease in an individual, the method comprising administering to the individual an effective amount of the compound of aspect 4.
11. A method of treating or preventing an inflammatory or fibrotic disease in an individual, the method comprising administering to the individual an effective amount of the compound of aspect 5.
12. A method of treating or preventing an inflammatory or fibrotic disease in an individual, the method comprising administering to the individual an effective amount of the compound of aspect 6.
13. The method of any one of aspects 7-12 in which the inflammatory or fibrotic condition is selected from the group consisting of fibrosis, idiopathic pulmonary fibrosis, chronic obstructive pulmonary disease, inflammatory pulmonary fibrosis, rheumatoid arthritis; rheumatoid spondylitis; osteoarthritis; gout; sepsis; septic shock; endotoxic shock; gram-negative sepsis; toxic shock syndrome; myofacial pain syndrome (MPS); Shigellosis; asthma; adult respiratory distress syndrome; inflammatory bowel disease; Crohn's disease; psoriasis; eczema; ulcerative colitis; glomerular nephritis; scleroderma; chronic thyroiditis; Grave's disease; Ormond's disease; autoimmune gastritis; myasthenia gravis; autoimmune hemolytic anemia; autoimmune neutropenia; thrombocytopenia; pancreatic fibrosis; chronic active hepatitis; hepatic fibrosis; renal disease; renal fibrosis, irritable bowel syndrome; pyresis; restenosis; cerebral malaria; stroke and ischemic injury; neural trauma; Alzheimer's disease; Huntington's disease; Parkinson's disease; acute and chronic pain; allergies; cardiac hypertrophy, chronic heart failure; acute coronary syndrome; cachexia; malaria; leprosy; leishmaniasis; Lyme disease; Reiter's syndrome; acute synoviitis; muscle degeneration, bursitis; tendonitis; tenosynoviitis; herniated, ruptured, or prolapsed intervertebral disk syndrome; osteopetrosis; thrombosis; silicosis; pulmonary sarcosis; bone resorption disease; cancer; Multiple Sclerosis, lupus; fibromyalgia; AIDS; herpes zoster virus infection, herpes simplex virus infection; influenza virus; Severe Acute Respiratory Syndrome (SARS); cytomegalovirus infection; and diabetes mellitus.
14. A method of modulating a stress activated protein kinase (SAPK) system, comprising contacting a compound with a p38 mitogen-activated protein kinase (MAPK),
   wherein the compound exhibits an IC₅₀ value in the range of about 10 pM to about 500 µM for inhibition of the p38γ MAPK; and
   wherein the compound exhibits an IC₅₀ value for inhibition of the p38α MAPK which is at least twofold higher than the IC₅₀ value for inhibition of the p38γ MAPK.
15. The method of aspect 14, wherein the compound exhibits an IC₅₀ value in the range of about 10 pM to about 5 µM for inhibition of the p38γ MAPK.
16. The method of aspect 14, wherein the compound exhibits an IC₅₀ value in the range of about 100 nM to about 500 µM for inhibition of the p38γ MAPK.
17. A method of modulating a stress activated protein kinase (SAPK) system, comprising contacting a compound with a p38 mitogen-activated protein kinase (MAPK),
   wherein the compound exhibits an IC₅₀ value in the range of about 10 pM to about 500 µM for inhibition of the p38γ MAPK; and
   wherein the compound exhibits an IC₅₀ value for inhibition of the p38α MAPK which is at least fivefold higher than the IC₅₀ value for inhibition of the p38γ MAPK.
18. The method of aspect 17, wherein the compound exhibits an IC₅₀ value in the range of about 10 pM to about 5 µM for inhibition of the p38γ MAPK.
19. The method of aspect 17, wherein the compound exhibits an IC₅₀ value in the range of about 100 nM to about 500 µM for inhibition of the p38γ MAPK.
20. A method of modulating a stress activated protein kinase (SAPK) system, comprising contacting a compound with a p38 mitogen-activated protein kinase (MAPK),
   wherein the compound exhibits an IC₅₀ value in the range of about 10 pM to about 500 µM for inhibition of the p38γ MAPK; and
   wherein the compound exhibits an IC₅₀ value for inhibition of the p38α MAPK which is at least tenfold higher than the IC₅₀ value for inhibition of the p38γ MAPK.
21. The method of aspect 20, wherein the compound exhibits an IC₅₀ value in the range of about 10 pM to about 5 µM for inhibition of the p38γ MAPK.
22. The method of aspect 20, wherein the compound exhibits an IC₅₀ value in the range of about 100 nM to about 500 µM for inhibition of the p38γ MAPK.
23. A method of modulating a stress activated protein kinase (SAPK) system, comprising contacting a compound with a p38 mitogen-activated protein kinase (MAPK),
   wherein the compound exhibits an IC₅₀ value in the range of about 10 pM to about 500 µM for inhibition of the p38γ MAPK; and
   wherein the compound exhibits an IC₅₀ value in the range of about 10 pM to about 1500 µM for inhibition of the p38α MAPK.
24. The method of aspect 23, wherein
   wherein the compound exhibits an IC₅₀ value in the range of about 10 pM to about 5 µM for inhibition of the p38γ MAPK; and
   wherein the compound exhibits an IC₅₀ value in the range of about 10 pM to about 5 µM for inhibition of the p38α MAPK.
25. The method of aspect 23, wherein
   wherein the compound exhibits an IC₅₀ value in the range of about 100 nM to about 500 µM for inhibition of the p38γ MAPK; and
   wherein the compound exhibits an IC₅₀ value in the range of about 100 nM to about 1500 µM for inhibition of the p38α MAPK.
26. The method of any one of aspects 14 to 25 in which the contacting is conducted at a SAPK-modulating concentration, wherein the compound exhibits an IC₅₀ value in the range of about 50 pM to about 1 µM for inhibition of the p38γ MAPK.
27. The method of any one of aspects 14 to 25 in which the contacting is conducted at a SAPK-modulating concentration, wherein the compound exhibits an IC₅₀ value in the range of about 50 pM to about 200 nM for inhibition of the p38γ MAPK.
28. The method of any one of aspects 14 to 27, further comprising administering the compound to a subject.
29. A method of treating or preventing a disease state in a subject, comprising
   identifying a subject at risk for or having an inflammatory or fibrotic condition; and
   administering a compound to the subject in an effective amount to treat or prevent the inflammatory or fibrotic condition;
   wherein the compound exhibits an IC₅₀ in the range of about 10 pM to about 500 µM for inhibition of the p38γ MAPK; and
   wherein the compound exhibits an IC₅₀ for inhibition of the p38α MAPK which is at least twofold higher than the IC₅₀ for inhibition of the p38γ MAPK.
30. The method of aspect 29, wherein the compound exhibits an IC₅₀ in the range of about 10 pM to about 5 µM for inhibition of the p38γ MAPK.
31. The method of aspect 29, wherein the compound exhibits an IC₅₀ in the range of about 100 nM to about 500 µM for inhibition of the p38γ MAPK.
32. A method of treating or preventing a disease state in a subject, comprising
   identifying a subject at risk for or having an inflammatory or fibrotic condition; and
   administering a compound to the subject in an effective amount to treat or prevent the inflammatory or fibrotic condition;
   wherein the compound exhibits an IC₅₀ in the range of about 10 µM to about 500 µM for inhibition of the p38γ MAPK; and
   wherein the compound exhibits an IC₅₀ for inhibition of the p38α MAPK which is at least fivefold higher than the IC₅₀ for inhibition of the p38γ MAPK.
33. The method of aspect 32, wherein the compound exhibits an IC₅₀ in the range of about 10 pM to about 5 µM for inhibition of the p38γ MAPK.
34. The method of aspect 32, wherein the compound exhibits an IC₅₀ in the range of about 100 nM to about 500 µM for inhibition of the p38γ MAPK.
35. A method of treating or preventing a disease state in a subject, comprising
   identifying a subject at risk for or having an inflammatory or fibrotic condition; and
   administering a compound to the subject in an effective amount to treat or prevent the inflammatory or fibrotic condition;
   wherein the compound exhibits an IC₅₀ in the range of about 10 pM to about 500 µM for inhibition of the p38γ MAPK; and
   wherein the compound exhibits an IC₅₀ for inhibition of the p38α MAPK which is at least tenfold higher than the IC₅₀ for inhibition of the p38γ MAPK.
36. The method of aspect 35, wherein the compound exhibits an IC₅₀ in the range of about 10 pM to about 5 µM for inhibition of the p38γ MAPK.
37. The method of aspect 35, wherein the compound exhibits an IC₅₀ in the range of about 100 nM to about 500 µM for inhibition of the p38γ MAPK.
38. The method of any of aspects 29-37, wherein the administered compound exhibits an IC₅₀ in the range of about 50 pM to about 1 µM for inhibition of the p38γ MAPK.
39. The method of any of aspects 29-37, wherein the administered compound exhibits an IC₅₀ in the range of about 50 pM to about 200 nM for inhibition of the p38γ MAPK.
40. A method of identifying a pharmaceutically active compound, comprising:
   providing a library of compounds;
   assaying a plurality of compounds from the library for inhibition of a p38α MAPK; and
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of the p38α MAPK which is at least tenfold higher than the IC₅₀ for inhibition of the p38γ MAPK.
41. The method of aspect 40, wherein the at least one compound exhibits an IC₅₀ in the range of about 10 pM to about 5 µM for inhibition of the p38γ MAPK.
42. The method of aspect 40, wherein the at least one compound exhibits an IC₅₀ in the range of about 100 nM to about 500 µM for inhibition of the p38γ MAPK.
43. A method of identifying a pharmaceutically active compound, comprising:
   providing a library of compounds;
   assaying a plurality of compounds from the library for inhibition of a p38α MAPK; and
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of the p38α MAPK which is at least fivefold higher than the IC₅₀ for inhibition of the p38γ MAPK.
44. The method of aspect 43, wherein the at least one compound exhibits an IC₅₀ in the range of about 10 pM to about 5 µM for inhibition of the p38γ MAPK.
45. The method of aspect 43, wherein the at least one compound exhibits an IC₅₀ in the range of about 100 nM to about 500 µM for inhibition of the p38γ MAPK.
46. A method of identifying a pharmaceutically active compound, comprising:
   providing a library of compounds;
   assaying a plurality of compounds from the library for inhibition of a p38α MAPK; and
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of the p38α MAPK which is at least twofold higher than the IC₅₀ for inhibition of the p38γ MAPK.
47. The method of aspect 46, wherein the at least one compound exhibits an IC₅₀ in the range of about 10 pM to about 5 µM for inhibition of the p38γ MAPK.
48. The method of aspect 46, wherein the at least one compound exhibits an IC₅₀ in the range of about 100 nM to about 500 µM for inhibition of the p38γ MAPK.
49. A method of identifying a pharmaceutically active compound, comprising:
   providing a library of compounds;
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of a p38α MAPK which is at least tenfold higher than an IC₅₀ for inhibition of the p38γ MAPK.
50. The method of aspect 49, wherein the at least one compound exhibits an IC₅₀ in the range of about 10 pM to about 5 µM for inhibition of the p38γ MAPK.
51. The method of aspect 49, wherein the at least one compound exhibits an IC₅₀ in the range of about 100 nM to about 500 µM for inhibition of the p38γ MAPK.
52. A method of identifying a pharmaceutically active compound, comprising:
   providing a library of compounds;
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of a p38α MAPK which is at least fivefold higher than an IC₅₀ for inhibition of the p38γ MAPK.
53. The method of aspect 52, wherein the at least one compound exhibits an IC₅₀ in the range of about 10 pM to about 5 µM for inhibition of the p38γ MAPK.
54. The method of aspect 52, wherein the at least one compound exhibits an IC₅₀ in the range of about 100 nM to about 500 µM for inhibition of the p38γ MAPK.
55. A method of identifying a pharmaceutically active compound, comprising:
   providing a library of compounds;
   assaying a plurality of compounds from the library for inhibition of a p38γ MAPK; and
   selecting at least one compound from the plurality of compounds, wherein the compound exhibits an IC₅₀ in the range of about 10 pM to about 500 µM for inhibition of the p38γ MAPK;
   wherein the compound exhibits an IC₅₀ for inhibition of a p38α MAPK which is at least twofold higher than an IC₅₀ for inhibition of the p38γ MAPK.
56. The method of aspect 55, wherein the at least one compound exhibits an IC₅₀ in the range of about 10 pM to about 5 µM for inhibition of the p38γ MAPK.
57. The method of aspect 55, wherein the at least one compound exhibits an IC₅₀ in the range of about 100 nM to about 500 µM for inhibition of the p38γ MAPK.
58. A method of identifying a pharmaceutically active compound, comprising:
   providing a library of compounds;
   assaying a plurality of compounds from the library for inhibition of a p38α MAPK; and
   selecting at least one compound from the plurality of compounds, wherein the compound exhibits an IC₅₀ in the range of about 10 pM to about 1500 µM for inhibition of the p38α MAPK; and
   wherein the compound exhibits an IC₅₀ for inhibition of the p38α MAPK which is at least tenfold higher than an IC₅₀ for inhibition of a p38γ MAPK.
59. The method of aspect 58, wherein the at least one compound exhibits an IC₅₀ in the range of about 10 pM to about 5 µM for inhibition of the p38γ MAPK.
60. The method of aspect 58, wherein the at least one compound exhibits an IC₅₀ in the range of about 100 nM to about 1500 µM for inhibition of the p38γ MAPK.
61. A method of identifying a pharmaceutically active compound, comprising:
   providing a library of compounds;
   assaying a plurality of compounds from the library for inhibition of a p38α MAPK; and
   selecting at least one compound from the plurality of compounds, wherein the compound exhibits an IC₅₀ in the range of about 10 pM to about 1500 µM for inhibition of the p38α MAPK; and
   wherein the compound exhibits an IC₅₀ for inhibition of the p38α MAPK which is at least fivefold higher than an IC₅₀ for inhibition of a p38γ MAPK.
62. The method of aspect 61, wherein the at least one compound exhibits an IC₅₀ in the range of about 10 pM to about 5 µM for inhibition of the p38γ MAPK.
63. The method of aspect 61, wherein the at least one compound exhibits an IC₅₀ in the range of about 100 nM to about 1500 µM for inhibition of the p38γ MAPK.
64. A method of identifying a pharmaceutically active compound, comprising:
   providing a library of compounds;
   assaying a plurality of compounds from the library for inhibition of a p38α MAPK; and
   selecting at least one compound from the plurality of compounds, wherein the compound exhibits an IC₅₀ in the range of about 10 pM to about 1500 µM for inhibition of the p38α MAPK; and
   wherein the compound exhibits an IC₅₀ for inhibition of the p38α MAPK which is at least twofold higher than an IC₅₀ for inhibition of a p38γ MAPK.
65. The method of aspect 64, wherein the at least one compound exhibits an IC₅₀ in the range of about 10 pM to about 5 µM for inhibition of the p38γ MAPK.
66. The method of aspect 64, wherein the at least one compound exhibits an IC₅₀ in the range of about 100 nM to about 1500 µM for inhibition of the p38γ MAPK.
67. The method of any of aspects 40-66 in which the contacting is conducted at a SAPK-modulating concentration, wherein the contacting compound exhibits an IC₅₀ in the range of about 50 pM to about 1 µM for inhibition of the p38γ MAPK.
68. The method of any of aspects 40-66 in which the contacting is conducted at a SAPK-modulating concentration, wherein the contacting compound exhibits an IC₅₀ in the range of about 50 pM to about 200 nM for inhibition of the p38γ MAPK.
69. The method of any of aspects 40 to 68 further comprising determining a mammalian toxicity of the selected compound.
70. The method of any of aspects 40 to 69 further comprising administering the selected compound to a test subject.
71. The method of aspect 70 in which the test subject has or is at risk for having an inflammatory or fibrotic condition.
72. The method of any one of aspects 14-71, wherein the compound is not selected from the group consisting of: 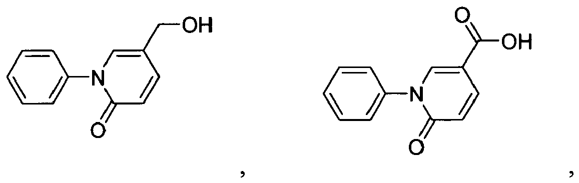 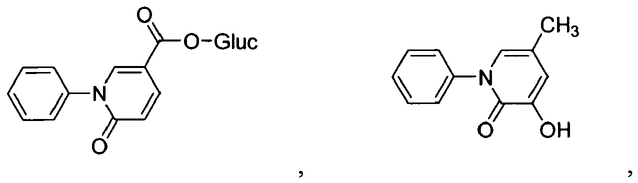 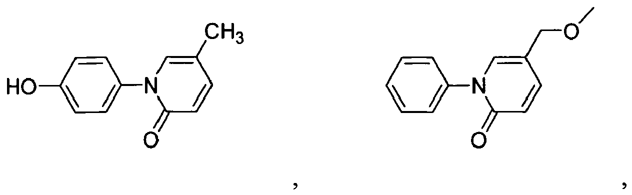 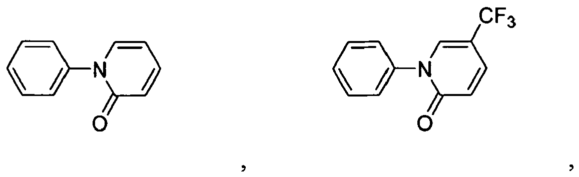 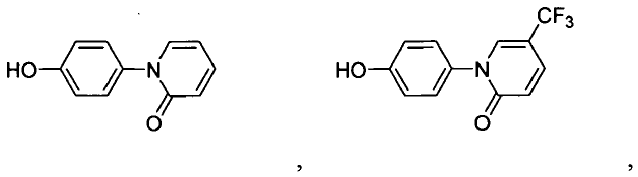 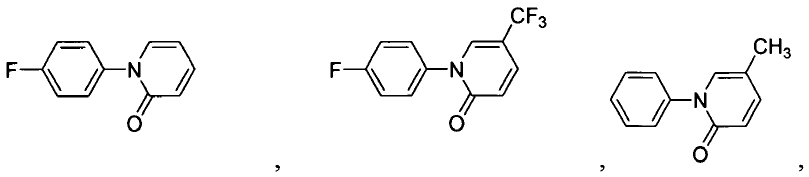 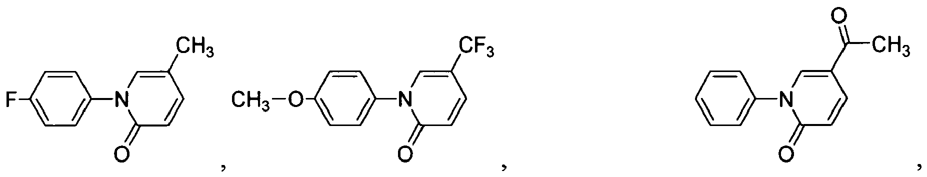 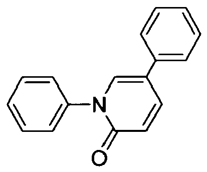 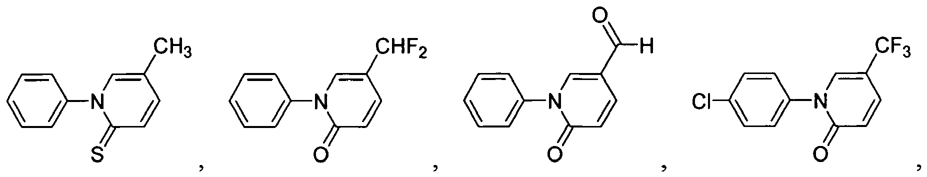 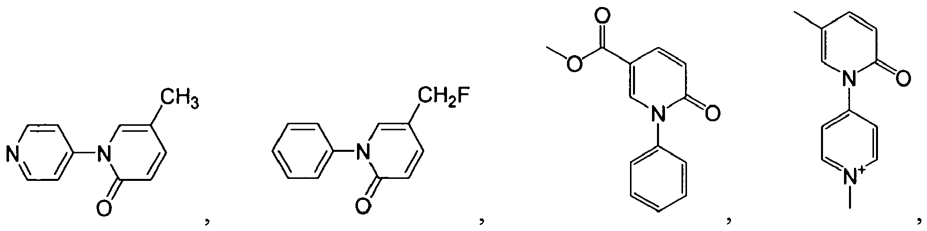 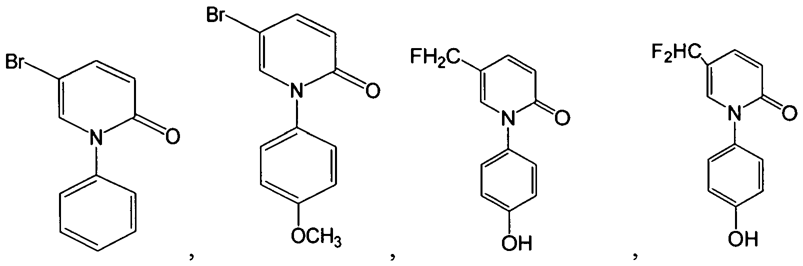 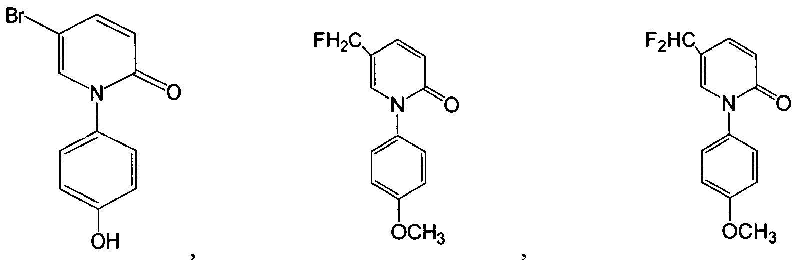 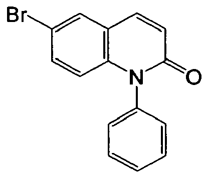 , and pirfenidone.
73. A pharmaceutical composition comprising:
   a) the compound of aspect 1 or aspect 2 or aspect 3 or aspect 4 or aspect 5 or aspect 6; and
   b) a pharmaceutically acceptable carrier.
74. A method of treating or preventing a disease or condition described herein, comprising administering to an individual an effective amount of the compound of any one of aspects 1 to 6.

## Claims

1. A compound of the formula: 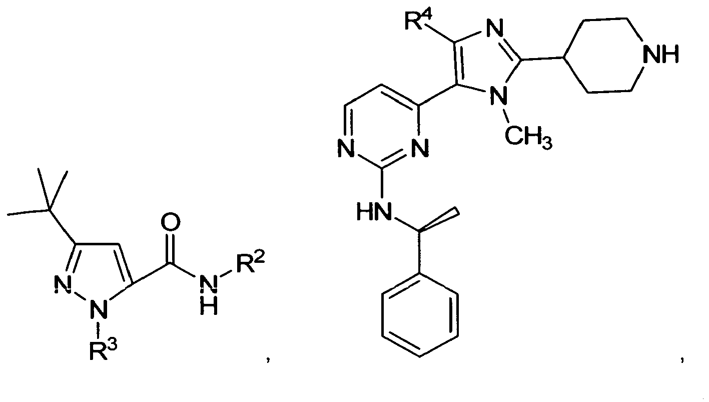 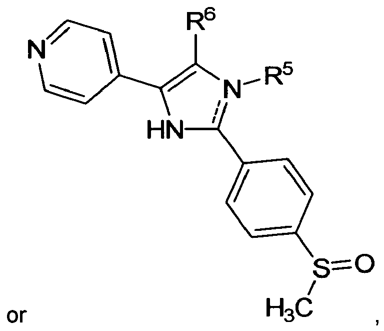 wherein
R² and R³ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl;
R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl,
R⁵ and R⁶ are each individually selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl wherein the compound is not
1-(5-tert-Butyl-2-p-tolyl-2H-pyrazol-3-yl)-3-[4-(2-morpholin-4-yl-ethoxy)naph-thalen-1-yl]urea (BIRB 796) or 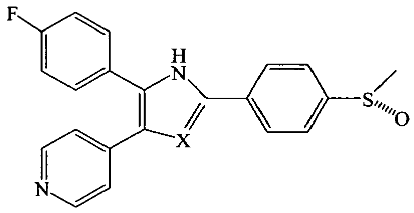 where X is N.

2. The compound of Claim 1 having a formula: 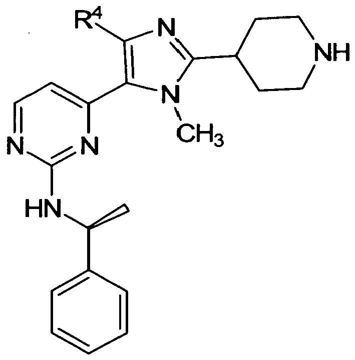 wherein, upon binding of the compound to p38γ, R⁴ is a group positioned not less than 5 A and not more than 25 A distant from a p38γ Met109 sulfhydryl moiety.

3. The compound of Claim 2 wherein R⁴ is selected from the group consisting of H, halo, cyano, nitro, hydroxy, optionally substituted C₁₋₆ alkyl, optionally substituted C₃₋₇ cycloalkyl, optionally substituted C₄₋₁₀ alkylcycloalkyl, optionally substituted C₂₋₆ alkenyl, optionally substituted C₁₋₆ alkoxy, optionally substituted C_{6 or 10} aryl, optionally substituted pyridinyl, optionally substituted pyrimidinyl, optionally substituted thienyl, optionally substituted furanyl, optionally substituted thiazolyl, optionally substituted oxazolyl, optionally substituted phenoxy, optionally substituted thiophenoxy, optionally substituted sulphonamido, optionally substituted urea, optionally substituted thiourea, optionally substituted amido, optionally substituted keto, optionally substituted carboxyl, optionally substituted carbamyl, optionally substituted sulphide, optionally substituted sulphoxide, optionally substituted sulphone, optionally substituted amino, optionally substituted alkoxyamino, optionally substituted alkyoxyheterocyclyl, optionally substituted alkylamino, optionally substituted alkylcarboxy, optionally substituted carbonyl, optionally substituted spirocyclic cycloalkyl, optionally substituted pyrazinyl, optionally substituted pyridazinyl, optionally substituted pyrrolyl, optionally substituted thiophenyl, optionally substituted thiazolyl, optionally substituted oxazolyl, optionally substituted imidazolyl, optionally substituted isoxazolyl, optionally substituted pyrazolyl, optionally substituted isothiazolyl, optionally substituted napthyl, optionally substituted quinolinyl, optionally substituted isoquinolinyl, optionally substituted quinoxalinyl, optionally substituted benzothiazolyl, optionally substituted benzothiophenyl, optionally substituted benzofuranyl, optionally substituted indolyl, and optionally substituted benzimidazolyl.

4. A pharmaceutical composition comprising the compound of any one of Claims 1 to 3 and a pharmaceutically acceptable carrier.

5. The compound of any one of Claims 1 to 3 for use in treating or preventing an inflammatory or fibrotic disease in an individual.

6. The compound for use according to Claim 5, wherein the inflammatory or fibrotic condition is selected from the group consisting of fibrosis, idiopathic pulmonary fibrosis, chronic obstructive pulmonary disease, inflammatory pulmonary fibrosis, rheumatoid arthritis; rheumatoid spondylitis; osteoarthritis; gout; sepsis; septic shock; endotoxic shock; gram-negative sepsis; toxic shock syndrome; myofacial pain syndrome (MPS); Shigellosis; asthma; adult respiratory distress syndrome; inflammatory bowel disease; Crohn's disease; psoriasis; eczema; ulcerative colitis; glomerular nephritis; scleroderma; chronic thyroiditis; Grave's disease; Ormond's disease; autoimmune gastritis; myasthenia gravis; autoimmune hemolytic anemia; autoimmune neutropenia; thrombocytopenia; pancreatic fibrosis; chronic active hepatitis; hepatic fibrosis; renal disease; renal fibrosis, irritable bowel syndrome; pyresis; restenosis; cerebral malaria; stroke and ischemic injury; neural trauma; Alzheimer's disease; Huntington's disease; Parkinson's disease; acute and chronic pain; allergies; cardiac hypertrophy, chronic heart failure; acute coronary syndrome; cachexia; malaria; leprosy; leishmaniasis; Lyme disease; Reiter's syndrome; acute synoviitis; muscle degeneration, bursitis; tendonitis; tenosynoviitis; herniated, ruptured, or prolapsed intervertebral disk syndrome; osteopetrosis; thrombosis; silicosis; pulmonary sarcosis; bone resorption disease; cancer; Multiple Sclerosis, lupus; fibromyalgia; AIDS; herpes zoster virus infection, herpes simplex virus infection; influenza virus; Severe Acute Respiratory Syndrome (SARS); cytomegalovirus infection; and diabetes mellitus.
